(19) **Europäisches Patentamt**
**European Patent Office**
**Office européen des brevets**

(11) **EP 3 429 457 B1**

(12) **EUROPÄISCHE PATENTSCHRIFT**

(45) Veröffentlichungstag und Bekanntmachung des Hinweises auf die Patenterteilung:
**13.05.2026 Patentblatt 2026/20**

(51) Internationale Patentklassifikation (IPC):
**A61B 5/12** (2006.01) **G16H 40/63** (2018.01)
**A61B 5/00** (2006.01)

(21) Anmeldenummer: **17712027.6**

(22) Anmeldetag: **14.03.2017**

(52) Gemeinsame Patentklassifikation (CPC):
**G16H 40/63; A61B 5/125; A61B 5/7235; A61B 5/7271;** A61B 5/7239

(86) Internationale Anmeldenummer:
**PCT/EP2017/000334**

(87) Internationale Veröffentlichungsnummer:
**WO 2017/157519 (21.09.2017 Gazette 2017/38)**

(54) **VERFAHREN ZUR AUTOMATISCHEN BESTIMMUNG EINER INDIVIDUELLEN FUNKTION EINER DPOAE-PEGELKARTE EINES MENSCHLICHEN ODER TIERISCHEN GEHÖRS**

METHOD FOR AUTOMATICALLY DETERMINING AN INDIVIDUAL FUNCTION OF A DPOAE LEVEL MAP OF HUMAN OR ANIMAL HEARING

PROCÉDÉ POUR DÉTERMINER DE MANIÈRE AUTOMATIQUE UNE FONCTION INDIVIDUELLE D'UNE CARTE DE NIVEAUX SELON LA MÉTHODE DE MESURE DES PRODUITS DE DISTORSION DES ÉMISSIONS OTO-ACOUSTIQUES (ÉOAPD) DE L'OUÏE CHEZ L'HOMME OU L'ANIMAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.03.2016 DE 102016003133**

(43) Veröffentlichungstag der Anmeldung:
**23.01.2019 Patentblatt 2019/04**

(73) Patentinhaber: **Eberhard-Karls-Universität Tübingen**
**72074 Tübingen (DE)**

(72) Erfinder:
• **DALHOFF, Ernst**
**72108 Rottenburg (DE)**
• **ZELLE, Dennis**
**72070 Tübingen (DE)**

(74) Vertreter: **Graf von Stosch Patentanwaltsgesellschaft mbH Triftstraße 5 80538 München (DE)**

(56) Entgegenhaltungen:
• M. L. WHITEHEAD ET AL: "Dependence of distortion-product otoacoustic emissions on primary levels in normal and impaired ears. II. Asymmetry in L 1 , L 2 space", THE JOURNAL OF THE ACOUSTICAL SOCIETY OF AMERICA, vol. 97, no. 4, 1 April 1995 (1995-04-01), New York, NY, US, pages 2359 - 2377, XP055378625, ISSN: 0001-4966, DOI: 10.1121/1.411960
• ZELLE DENNIS ET AL: "Level dependence of the nonlinear-distortion component of distortion-product otoacoustic emissions in humansa)", THE JOURNAL OF THE ACOUSTICAL SOCIETY OF AMERICA, AMERICAN INSTITUTE OF PHYSICS FOR THE ACOUSTICAL SOCIETY OF AMERICA, NEW YORK, NY, US, vol. 138, no. 6, 8 December 2015 (2015-12-08), pages 3475 - 3490, XP012203260, ISSN: 0001-4966, [retrieved on 19010101], DOI: 10.1121/1.4936860
• DAVID M. MILLS: "Interpretation of distortion product otoacoustic emission measurements. I. Two stimulus tones", THE JOURNAL OF THE ACOUSTICAL SOCIETY OF AMERICA, vol. 102, no. 1, 1 July 1997 (1997-07-01), New York, NY, US, pages 413 - 429, XP055378615, ISSN: 0001-4966, DOI: 10.1121/1.419763

**Beschreibung**

Die vorliegende Erfindung betrifft ein Verfahren zur automatischen Bestimmung einer individuellen Funktion einer DPOAE-Pegelkarte eines menschlichen oder tierischen Gehörs gemäß Anspruch 1 sowie ein System zur Durchführung dieses Verfahrens gemäß dem nebengeordneten Anspruch. Die vorliegende Erfindung betrifft insbesondere ein Verfahren mit einzelnen Merkmalen des Anspruchs 1 sowie ein System zur Durchführung dieses Verfahrens mit einzelnen Merkmalen des nebengeordneten Anspruchs.

Das Hörsystem kann als eine Kette von aufeinanderfolgenden Signalverarbeitungsblöcken angesehen werden. Diese werden durchlaufen, bevor im Cortex die komplexere Wahrnehmung des Hörens entsteht. Die ersten Blöcke der Signalverarbeitungskette sind das äußere Ohr (Ohrmuschel & Gehörgang), das Mittelohr (Gehörknöchelchen mit der Fußplatte als Grenze zu den Flüssigkeiten des Innenohrs), und das flüssigkeitsgefüllte Innenohr. Die allermeisten Hörschäden entstehen im Innenohr. Darunter fällt auch die Altersschwerhörigkeit, die im Mittel im Alter von 60-70 Jahren zu 25 dB Hörverlust bei Frauen bzw. 35 dB bei Männern im Frequenzbereich ab 4 kHz führt. Sie ist dominiert durch eine Beeinträchtigung des sog. cochleären Verstärkers, der im gesunden Zustand die eingehenden Schallwellen um den Faktor 300-1000 verstärkt, bevor sie von den inneren Haarzellen und deren Synapsen in neurale Signale umgewandelt werden.

Seit etwa 1980 ist die Existenz des cochleären Verstärkers sukzessive nachgewiesen worden, und dabei spielte die Entdeckung der otoakustischen Emissionen (OAE) durch David T. Kemp eine zentrale Rolle. Diese sind Töne, die vom aktiven Verstärker als Nebenprodukt erzeugt und durch das Mittelohr rückwärts zum Gehörgang übertragen werden. Dort können sie mit empfindlichen Miniaturmikrophonen gemessen werden.

Eine Form der OAE sind die Distorsionsprodukt-otoakustischen Emissionen (DPOAE), bei denen üblicherweise zwei Primärtöne mit den Frequenzen $f_1$ und $f_2$ und den Pegeln $L_1$ und $L_2$ präsentiert werden. Die nichtlineare Kennlinie der mechanoelektrischen Transduktion der Ionenkanäle der äußeren Haarzellen, die das hauptsächliche Motorelement des cochleären Verstärkers beim Menschen und den Säugetieren allgemein darstellen, führt zu zahlreichen Distorsionsprodukten. Das am einfachsten messbare Distorsionsprodukt und daher in diagnostischen Anwendungen bevorzugte ist dasjenige bei $f_{dp} = 2f_1 - f_2$ mit $f_2 > f_1$ und einem optimalen Frequenzverhältnis von etwa $\dfrac{f_2}{f_1} = 1,2$. Heutzutage werden üblicherweise DPOAE so angeregt, dass bei dem cochleären Abbildungsort des zweiten Primärtons beide Anregungsfrequenzen zu möglichst gleichgroßen Schwingungsamplituden der Basilarmembran führen, und dementsprechend werden diagnostische Schlüsse aus DPOAE-Befunden bei der Frequenz und entsprechend dem Anregungspegel des zweiten Primärtons $\{f_2, L_2\}$ interpretiert. DPOAE-Messungen können z.B. nach dem in der DE 102014108663 beschriebenen Verfahren bei verschiedenen Frequenzen durchgeführt und interpretiert werden.

Ein bekanntes Verfahren zur Bestimmung der Hörschwelle, bzw. genauer der Schwelle des cochleären Verstärkers des Innenohres, beruht auf der Bestimmung der Schwelle, ab der eine Emission, insbesondere eine Distorsionsprodukt-otoakustische Emission (DPOAE), messbar ist, eine Technik, die zuerst bei Menschen eingesetzt wurde [B. P. Kimberley and D. A. Nelson., J. Otolaryngol., 18(7): 365-369, 12 1989; D. A. Nelson and B. P. Kimberley, J.Speech Hear.Res., 35(5):1142-1159, 10 1992].

Hierzu wird üblicherweise eine Wachstumsfunktion gemessen, bspw. die Emissionen mit Anregungspegeln von $L_2$=60 dB SPL absteigend in 5 dB-Schritten, bis ein gewisses SNR (Signal-to-Noise Ratio) nicht mehr erreicht wird. Der Anregungspegel, bei dem gerade noch das geforderte SNR erreicht wird, wird dann als DPOAE-Schwelle bezeichnet.

Nach Boege und Janssen [P. Boege and T. Janssen, J. Acoust. Soc. Am., 111(4): 1810-1818, 04, 2002] werden die Werte der Wachstumsfunktion semilogarithmisch aufgetragen, das heißt, die DPOAE werden linear als Schalldruck in Einheiten von [μPa] auf der Ordinate aufgetragen, gegen den Anregungsschalldruck des zweiten Primärtons $L_2$ in der logarithmischen Einheit von [dB SPL].

Mit sogenannten optimalen Anregungsschalldrücken [P. Kummer, T. Janssen, P. Hulin, and W. Arnold. Optimal L1-L2 primary tone level separation remains independent of test frequency in humans. Hear.Res., 146(1-2):47-56, 08 2000] erhält man typischerweise eine lineare Wachstumsfunktion, die man mittels linearer Regression bis zur Abszisse extrapolieren kann. In diesem Fall wird der extrapolierte Schnittpunkt von Wachstumsfunktion und der Abszisse als geschätzte DPOAE-Schwelle (auch: "estimated distorsion product threshold", EDPT) bezeichnet.

Im allgemeinen korreliert diese DPOAE-Schwelle gut mit den psychoakustisch gemessenen Schwellen [P. Boege and T. Janssen, J. Acoust. Soc. Am., 111(4): 1810-1818, 04, 2002, M. P. Gorga et. al., J. Acoust. Soc. Am., 113(6): 3275-3284, 06, 2003.], weist aber eine unbefriedigend hohe Standardabweichung und in einzelnen Fällen eine Abweichung von bis zu 40 dB auf [N. Schmuziger et. al., J. Acoust. Soc. Am., 119(4): 1937-1939, 04, 2006]. Dies liegt teilweise daran, daß durch Interferenz beider Quellbeiträge, die bei der Messung konventioneller, kontinuierlich dargebotener DPOAE zur sogenannten DPOAE-Feinstruktur führen, Meßfehler auftreten, die gerade beim Extrapolationsverfahren zu gravierenden Fehlschätzungen führen [E. Dalhoff, A. Vetesnik, D. Turcanu, and A. W. Gummer. Schall- und Geschwindigkeits-DPOAE: Technologie, Methodik und Perspektiven. HNO, 58(6):543-555, 06 2010]. Um die beiden interferierenden Quellbeiträge zu separieren, können verschiedene Methoden eingesetzt werden, unter anderem auch diejenige der gepulsten DPOAE [D. Zelle, A. W. Gummer, and E. Dalhoff. Extraction of otoacoustic distortion product sources using pulse basis functions. J.Acoust.Soc.Am., 134(1):EL64-EL69, 07 2013]. Eine andere Ursache für Fehlschätzungen liegt darin, dass die optimalen Anregungspegel individuell variieren.

In [M. L. Whitehead, B. B. Stagner, M. J. Mccoy, B. L. Lonsbury-martin, G. K. Martin. Dependence of distortion-product otoacoustic emissions on primary levels in normal and impaired ears. II. Asymmetry in L 1, L 2 space. The Journal of the Acoustical Society of America, 97(4), 1995] wird der Zusammenhang zwischen $L_1/L_2$-Pegeln und DPOAE untersucht.

Untersuchungen der Anmelder mit gepulsten DPOAE haben ergeben, dass die für eine Studienpopulation optimalen Parameter für die Anregungspegel im Einzelfall von den individuell optimalen Anregungsparametern deutlich abweichen, mit folgenden unerwünschten Begleiterscheinungen: 1) Es werden DPOAE von deutlich niedrigeren Amplituden gemessen, was in den üblichen Realisierungen von automatisierten Messverfahren dazu führt, dass länger gemessen werden muss, um das geforderte SNR zu erreichen; 2) der global optimale Anregungspfad (global optimal: als Gruppenmittelwert bestimmt) liegt so, dass er im individuellen Fall zu einer deutlichen Verformung der Wachstumsfunktion und damit zu einer Fehlschätzung bei der Extrapolation führt, die auf der Annahme einer linearen Funktion basiert.

Es ist eine Aufgabe der vorliegenden Erfindung, ein Verfahren bereitzustellen, das gegenüber dem Stand der Technik verbessert ist und das die individuellen Parameter eines menschlichen oder tierischen Gehörs einbezieht. Eine einfache Handhabung ist ebenfalls wünschenswert. Diese Aufgabe wird gelöst durch ein Verfahren zur automatischen Bestimmung einer individuellen Funktion einer DPOAE-Pegelkarte gemäß Anspruch 1.

Das erfindungsgemäße Verfahren kann für konventionelle, d.h. quasi kontinuierlich gemessene DPOAE eingesetzt werden. Bevorzugt wird es mit einer Methode kombiniert, die Artefakte aufgrund der Interferenz von zwei verschiedenen Quellbeiträgen einer DPOAE unterdrückt, wie z.B. ein Verfahren mit gepulsten DPOAE gemäß DE 102014108663 A1.

Durch das erfindungsgemäße Verfahren zur automatischen Bestimmung einer individuellen Funktion einer DPOAE-Pegelkarte werden Fehler bei der Extrapolation der Wachstumsfunktionen vermieden, die in den Verfahren zur Messung der Distorsionsproduktschwelle nach dem vorbeschriebenen Stand der Technik prinzipbedingt vorkommen.

Ferner werden gegenüber den bekannten Verfahren zusätzliche Daten erhalten, die dann für eine Diagnose zur Verfügung stehen. Neben der Distorsionsproduktschwelle $L_{edpt}$ und der Steigung der Wachstumsfunktion werden bei dem erfindungsgemäßen Verfahren auch Daten zur Frequenzauflösung und Kompression der zugrundeliegenden Wanderwellen und dem Schalleitungsverlust erfasst. Der generelle Vorteil der neuen Methode besteht also darin, daß bei gleichem oder sogar geringerem Zeitaufwand für die Meßpunkte vier statt im Stand der Technik zwei Informationen erhalten werden, und daß Schätzfehler bei den bisher erhaltenen Parametern (der Distorsionsproduktschwelle $L_{edpt}$ und der Steigung der Wachstumsfunktion) reduziert werden.

In einer vorteilhaften Weiterbildung der Erfindung weist das erste Pegelpaar $\{L_1^{(1)}, L_2^{(1)}\}$ einen Pegel $L_1$ von 67 $\pm$ 10 dB und einen Pegel $L_2$ von 57 $\pm$ 10 dB auf. Diese Pegel von $L_1$ und von $L_2$ haben sich als besonders günstige Anfangspegel erwiesen. Bei Normalhörenden befinden sich diese Anregungspegel nämlich noch in dem Bereich, bis zu welchem die Pegelkarte näherungsweise linear ansteigt, und selbst bei Hörverlusten bis ca. 40 dB kann immer noch ein DPOAE bei diesen Pegeln gemessen werden. Man erhält also in den meisten Fällen Werte, die für die Erfassung der Pegelkarte valide sind.

Die Modellfunktion definiert einen linear ansteigenden Grat, dem linear miteinander verknüpfte $\{L_1^{(G)}, L_2^{(G)}\}$ Pegelpaare zugeordnet sind (wobei "G" der Index für "dem Grat zugeordnet" ist). Erfindungsgemäß liegen mindestens die Hälfte der gemessenen Pegelpaare $\{L_1^{(i)}, L_2^{(i)}\}$ um mindestens 5 dB zu beiden Seiten abseits der dem Grat zuge-ordneten $\{L_1^{(G)}, L_2^{(G)}\}$ Pegelpaare (wobei "i" der Index der Messung von 1 bis n ist).

In einer weiteren vorteilhaften Ausbildung der Erfindung werden die unterschiedlichen Pegelpaare $\{L_1^{(i)}, L_2^{(i)}\}$ in einer Sequenz präsentiert, die für jedes Individuum identisch ist. Durch dieses stark vereinfachte und vereinheitlichte (starre) Verfahren wird zwar die Annäherung an die individuelle Funktion einer Pegelkarte etwas weniger genau, jedoch ist dieses Verfahren in der Durchführung sehr schnell.

Von Vorteil kann es ferner sein, wenn die vordefinierten, unterschiedlichen Pegelpaare $\{L_1, L_2\}$ in einer Sequenz präsentiert werden, die eine Anzahl von k Subsequenzen aufweist, deren Pegelpaare $\{L_1, L_2\}$ im Wesentlichen quer zu den linear miteinander verknüpften und dem Grat zugeordneten Pegelpaaren $\{L_1^{(G)}, L_2^{(G)}\}$ liegen. Durch die Einschaltung der Subsequenzen kann der Grat an mehreren Stellen abgetastet werden, was die Genauigkeit der Bestimmung der individuellen Funktion der DPOAE-Pegelkarte erhöht.

In einer günstigen Weiterbildung der Erfindung ist $n \geq 5$ und $\leq 12$, vorzugsweise $6 \leq n \leq 8$. Durch die kleine Anzahl an vorgesehenen Messungen wird eine kurze Messdauer erzielt bei gleichzeitig guter Erfassung der individuellen Funktion der DPOAE-Pegelkarte.

Vorteilhaft ist die Anzahl der Subsequenzen $k \geq 2$ und $\leq 5$, wodurch eine gute Abtastung des Grats der DPOAE Pegelkarte erreicht wird.

Weiter vorteilhaft ist es, wenn die einem ersten vordefinierten Pegelpaar $\{L_1^{(1)}\ L_2^{(1)}\}$ nachfolgenden Pegelpaare $\{L_1^{(2...n_k)},\ L_2^{(2...n_k)}\}$ einer Subsequenz mit $n_k$ Messungen über eine Funktion

$$\{L_1^{(i)},\ L_2^{(i)}\} = \{L_1^{(i-1)} +\ \mu\ \cdot \Delta L_1,\ L_2^{(i-1)} +\ \mu\ \cdot \Delta L_2\}$$

aus dem jeweils vorhergehenden Pegelpaar $L_1^{(i-1)},\ L_2^{(i-1)}$ bestimmt werden, wobei $\mu = \pm\ 1$, insbesondere $+1$ ist, und $\Delta L_1$, $\Delta L_2$ ein Pegelabstand von zwei aufeinanderfolgenden Pegelpaaren ist und Werte von $\Delta L_1 = 4$ bis 14 dB, vorzugsweise von 6 bis 10 dB, und $\Delta L_2 = 0$ bis -2,78 dB, vorzugsweise $\Delta L_2 = -1{,}52$ bis $-2{,}78$ dB aufweist. Der Faktor $\mu$ legt dabei die Suchrichtung (zu kleineren oder größeren $L_1$-Meßpegeln hin) quer zum Grat fest.

In einer günstigen Weiterbildung des Verfahrens, wird, wenn das erste Pegelpaar $\{L_1^{(1)}, L_2^{(1)}\}$ und das zweite Pegelpaar $\{L_1^{(2)}, L_2^{(2)}\}$ zwei DPOAE mit $p_{dp,l}(1...2)$ produziert, die einen Rauschabstand von je $>=4$ dB, vorzugsweise $>=10$ dB aufweisen, der Pegel eines nachfolgenden dritten Pegelpaars $\{L_1^{(3)},\ L_2^{(3)}\}$ wenigstens um $\Delta L_1 \geq 4$ dB unterschiedlich eingestellt als der Pegel des vorhergehenden Pegelpaars $\{L_1^{(2)}, L_2^{(2)}\}$, wenn $p_{dp,l}(2) - p_{dp,l}(1) > 0$, und wird andererseits der Pegel eines nachfolgendes Pegelpaars $\{L_1^{(3)},\ L_2^{(3)}\}$ wenigstens um $\Delta L_1 \leq -4$ dB unterschiedlich eingestellt als der Pegel des ersten Pegelpaars $\{L_1^{(1)}, L_2^{(1)}\}$, wenn $p_{dp,l}(2) - p_{dp,l}(1) \leq 0$. Mit dieser Vorgehensweise wird erreicht, daß wenigstens ein Punkt links und ein Punkt rechts des Grates und dazwischenliegend ein Punkt in der Nähe des Grates gemessen wird.

In einer vorteilhaften Weiterbildung des Verfahrens wird, wenn das erste Pegelpaar $\{L_1^{(1)},\ L_2^{(1)}\}$ keine DPOAE mit $p_{dp,l}(1)$ produziert, die einen Rauschabstand von $>=4$ dB, vorzugsweise $>=10$ dB aufweisen, in derselben Suchrichtung weiterverfahren, bis entweder der maximale bzw. minimale Anregungspegel $L_1^{(i)}$ erreicht ist, oder eine Gruppe von drei validen DPOAE mit $p_{dp,l}(i...i+2)$ produziert wurde, die einen Rauschabstand von je $>=4$ dB, vorzugsweise $>=10$ dB aufweisen. Hiermit wird gegenüber einem starren Verfahren erreicht, daß der Grat auch dann aufgefunden wird, wenn er deutlich neben der für Normalhörende zu erwartenden Position liegt, wie es etwa bei Schallleitungsstörungen der Fall sein kann.

In einer vorteilhaften Weiterbildung des Verfahrens, wird, wenn in der ersten Subsequenz nach Messung bei $i$ Anregungspegelpaaren keine Gruppe von drei validen DPOAE produziert wird, die einen Rauschabstand von je $>=4$ dB, vorzugsweise $>=10$ dB aufweisen, eine weitere Subsequenz mit höherem Pegelpaar $\{L_1^{(i+1)}, L_2^{(i+1)}\}$ gestartet, wobei das Startpegelpaar für die erneute Subsequenz auf $L_2^{(i+3)} = L_2^{(1)} + 20 \pm 10\ dB$, $L_1^{(i+3)} = L_1^{(1)} + 20 \pm 10\ dB$ eingestellt wird. Der Pegel wird vorzugsweise auf den technisch maximal erreichbaren bzw. sinnvollen Pegel begrenzt. Dieser maximale Pegel kann z.B. bei 75-85 dB SPL Schalldruck liegen. Durch diese Vorgehensweise, können auch stark vom Durchschnitt abweichende individuelle Pegelkarten bzw. deren Funktion noch ermittelt werden.

In einer vorteilhaften Ausführungsform des erfindungsgemäßen Verfahrens, wird, nach der Erfassung der DPOAE von wenigstens 3 Pegelpaaren $\{L_1^{(1..3)},\ L_2^{(1..3)}\}$, die vorzugsweise einer Subsequenz zugeordnet sind, aus diesen 3 Pegelpaaren $\{L_1^{(1..3)},\ L_2^{(1..3)}\}$ die Lage des Grates $\{L_1^{(G)},\ L_2^{(G)}\}$ längs der durch die 3 Pegelpaare gebildeten Linie ermittelt und im Anschluss daran ein viertes Pegelpaar $\{L_1^{(4)},\ L_2^{(4)}\}$ präsentiert, welches in einem vorgegebenen Abstand gratabwärts liegt, wobei der Gruppenmittelwert der Gratrichtung, $\varphi$ verwendet wird, und wobei anhand der aus den vier präsentierten Pegelpaaren $\{L_1^{(1..4)},\ L_2^{(1..4)}\}$ bestimmten DPOAE eine Steigung m des linearen Grats der Pegelkarte ermittelt wird.

Vorzugsweise wird, wenn in der ersten oder zweiten Subsequenz eine Gruppe von drei validen DPOAE mit $p_{dp,l}^{(i-2...i)}$ produziert wird, die einen Rauschabstand von je >=4 dB, vorzugsweise >=10 dB aufweisen, durch automatische Anpassung einer geeigneten Berechnungsfunktion an die zugehörigen DPOAE $p_{dp,l}^{(i-2...i)}$ das Pegelpaar unterhalb des Grates $\{L_1^{(G)},\ L_2^{(G)}\} = \{L_1^{(i-2)} + \varepsilon \cdot \Delta L_1, L_2^{(i-2)} + \varepsilon \cdot \Delta L_2\}$ bestimmt, wobei $\varepsilon$ so berechnet wird, daß $p_{dp,l}\left(L_1^{(G)},\ L_2^{(G)}\right)$ ein Maximum bildet, und davon ausgehend ein viertes Pegelpaar $\{L_1^{(i+1)},\ L_2^{(i+1)}\}$ präsentiert wird, mit einer Funktion $\{L_1^{(i+1)},\ L_2^{(i+1)}\} = \{L_1^{(i)} + \Delta L_1, L_2^{(i)} + \Delta L_2\}$, wobei $\Delta L_2$ = -15 $\pm$ *10 dB* eingestellt wird, und das Pegelpaar vorzugsweise auf der Projektion des erwarteten Grates auf die $L_1, L_2$-Ebene, d.h. mit $\Delta L_1/\Delta L_2 \approx 0.51 \pm 0.15$ eingestellt wird, und wobei anhand der aus den vier präsentierten Pegelpaaren $L_1^{(i-2...i+1)},\ L_2^{(i-2...i+1)}$ bestimmten DPOAE die Steigung m des näherungsweise linearen Grats der Pegelkarte ermittelt wird.

Anhand der ermittelten Steigung m des linearen Grats der Pegelkarte können z.B. wenigstens zwei, vorzugsweise drei weitere Pegelpaare $L_1^{(i+1...i+3)},\ L_2^{(i+1...i+3)}$ automatisch definiert werden, deren Anregungspegel in einer Subsequenz formiert werden, und die aufgrund der bereits bekannten Lage und Steigung des Grates so bestimmt werden, dass erwartet werden kann, valide DPOAE innerhalb einer Messzeit von je $t_m \leq 40$ s zu messen, wozu eine Modellfunktion an die vorzugsweise vier bereits valide gemessenen DPOAE angepasst wird, und dann in der Modellfunktion die letzten zwei oder drei Pegelpaare so bestimmt werden, dass die erwarteten DPOAE-Pegel vorzugsweise bei $p_{dp,l}^{(i+1...i+3)},\ p_{dp,l}^{(i+1...i+3)} \geq 10\ \mu\text{Pa}$ liegen.

Vorzugsweise werden die Pegelpaare $\{L_1^{(1-n)},\ L_2^{(1-n)}\}$ gepulst präsentiert, wobei jeder einzelne Puls mit einer Dauer $T_D$ von 2 bis 40 ms präsentiert wird. Durch die Verwendung einer solchen gepulsten Präsentation kann der Einfluss der beiden Quellbeiträge einer DPOAE unterdrückt bzw. separiert werden.

In einer vorteilhaften Fortbildung des Verfahrens, werden die Pegelpaare $\{L_1^{(1..n)},\ L_2^{(1..n)}\}$ in Blöcken aus mehreren, zeitlich nacheinander in Pulsen präsentierten Pegelpaaren $\{L_1^{(1..n)},\ L_2^{(1..n)}\}$ präsentiert, wobei zeitlich direkt aufeinanderfolgende Pegelpaare $\{L_1^{(1..n)}, L_2^{(1..n)}\}$ unterschiedliche Anregungsfrequenzen $\{f_2, f_1\}$ aufweisen. In einem Block folgen also auf ein erstes gepulst präsentiertes Pegelpaar mit $\{f_{2,1}, f_{1,1}\}$ ein zweites Pegelpaar mit unterschiedlichen Frequenzen $\{f_{2,2}, f_{1,2}\}$ und ggf. weitere mit $\{f_{2,m}, f_{1,m}\}$, wobei das Frequenzverhältnis immer nahe bei $f_{2,m}/f_{1,m}$ = 1,2 gehalten wird. Mehrere Blöcke mit Zeit-Frequenz-verschränkten Pulspaaren können gemittelt werden, bevor eine Auswertung erfolgt. Durch diese Maßnahme wird es möglich, die Zeit, in der die Pulsantwort auf eine Präsentation bei einem Frequenzpaar abklingt, zu nutzen, um bei einer anderen Frequenz zu messen, und so die Messzeit gegenüber einer hinsichtlich der gewünschten Messfrequenzen rein sequentiellen Vorgehensweise zu reduzieren.

Vorteilhaft wird in einem Verfahrensschritt, die ermittelte individuelle Funktion einer DPOAE-Pegelkarte und deren Parameter von der Rechnereinheit in einem nicht-flüchtiger Speicher abgespeichert. Ebenfalls können die ermittelten Rohdaten von der Rechnereinheit in dem nicht-flüchtiger Speicher abgespeichert werden. Die abgespeicherten Daten können von der Rechnereinheit zur kontinuierlichen Erweiterung des, der Modellfunktion einer Pegelkarte zugrundeliegenden Datensatzes verwendet werden.

Die gestellte Aufgabe wird ebenfalls noch durch ein System zur Durchführung des Verfahrens gelöst, wobei das System beinhaltet: eine Rechnereinheit, einen Arbeitsspeicher, einen nicht-flüchtigen Speicher zur Speicherung einer Modellfunktion $p_{dp,M}$ = *f(L1,L2)* und Modellparametern der Modellfunktion, wenigstens ein über die Rechnereinheit angesteuertes Tonausgabemittel zur Präsentation von Tönen an ein Individuum, wenigstens ein mit der Rechnereinheit verbundenes Tonaufnahmemittel zum Erfassen von DPOAE aus dem Ohr des Individuums. Ein Vorteil des neuen Systems liegt darin, daß bei gleichem oder sogar geringerem Zeitaufwand für die Messungen vier statt im Stand der Technik zwei Informationen erhalten werden, und daß Schätzfehler bei den bisher erhaltenen Parametern (der Distorsionsproduktschwelle $L_{edpt}$ und der Steigung der Wachstumsfunktion) reduziert werden.

Vorteilhaft ist, daß wenigstens ein Tonausgabemittel einen Lautsprecher mit einer hochlinearen Charakteristik aufweist, wodurch bei gleichzeitigem Abspielen zweier Töne $f_1, f_2$ keine Distorsion entsteht und ein Lautsprecher für die Präsentation beider Töne ausreichend ist.

Vorteilhaft ist ein Ausgabemittel zur Ausgabe der individuellen Funktion einer DPOAE-Pegelkarte vorgesehen, wie z.B. eine Anzeigeeinheit, Monitor, Display oder wie ein Drucker oder eine Schnittstelle zur Datenübertragung an eine externe Anzeigeeinheit, Monitor, Display oder Drucker etc., über die eine ermittelte individuellen Funktion einer DPOAE-Pegelkarte eines menschlichen oder tierischen Gehörs und deren Parameter von dem System ausgegeben werden und einem Nutzer zugänglich gemacht werden können.

Ferner ist vorteilhaft ein nicht-flüchtiger Speicher zur Speicherung der ermittelten individuellen Funktion einer DPOAE-Pegelkarte und deren Parameter vorgesehen.

In den nachfolgenden Figuren wird die Erfindung anhand von Ausführungsbeispielen näher erläutert. Es zeigen:

Figur 1    Ein System zur automatischen Bestimmung einer individuellen Funktion einer DPOAE-Pegelkarte;

Figur 2    Eine Modellfunktion deren dreidimensionaler Graph einer Modellpegelkarte entspricht;

Figur 3    erfindungsgemäße Schritte eines Verfahrens zur automatischen Bestimmung einer individuellen Funktion einer DPOAE-Pegelkarte;

Figur 4    eine Detaillierung des verfahrensgemäßen Schrittes nach der Markierung IV in Figur 3.

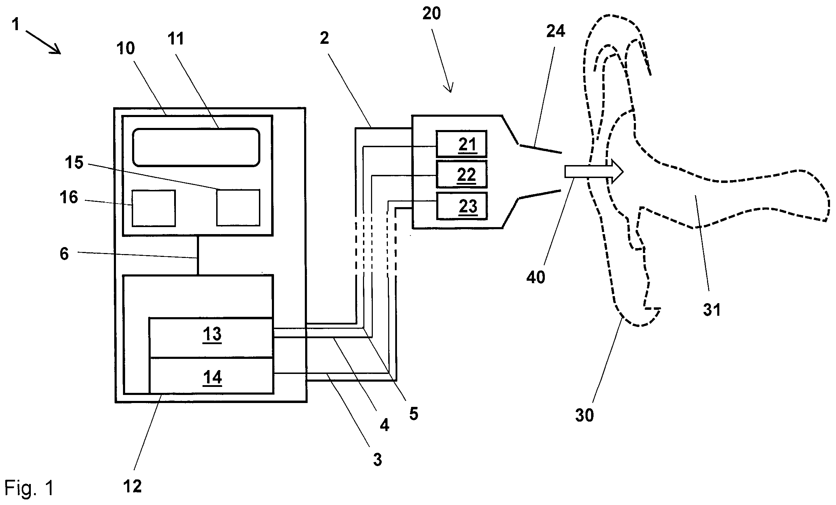

In Figur 1 ist ein System zur automatischen Bestimmung einer individuellen Funktion einer DPOAE-Pegelkarte eines menschlichen oder tierischen Gehörs in einer möglichen erfindungsgemäßen Ausgestaltung dargestellt. Zu dem System 1 gehören eine an einem Ohr positionierbare Sonden-Einheit 20, insbesondere eine OAE-Sonde, und eine Rechnereinheit 10. Die Sonden-Einheit weist eine Sondenspitze 24 auf, die in den Gehörgang eines Ohres einführbar ist. In der Sonden-Einheit 20 ist ein Tonaufnahmemittel 23, wie z.B. ein Mikrofon, angeordnet, welches zur Aufnahme von aus dem Gehörgang kommenden Tönen eingerichtet ist. In der Sonden-Einheit 20 sind ferner ein erstes und ein zweites Tonausgabemittel 21 und 22 vorgesehen, die als f1-Schallgeber (Tonausgabemittel 21) und als f2-Schallgeber (Tonausgabemittel 22) fungieren. Die Tonausgabemittel 21, 22 können dabei z. B. als Lautsprecher ausgebildet sein. Es kann auch nur ein Tonausgabemittel bzw. nur ein Lautsprecher vorgesehen sein, das/der zum gleichzeitigen Abspielen zweier Töne $f_1$, $f_2$ eingerichtet ist und insbesondere eine hochlineare Charakteristik besitzt. Die Sonden-Einheit 20 ist z.B. über eine Kabelverbindung 2 mit der Steuereinheit, die die Rechnereinheit 10 beinhaltet, verbunden. In der Kabelverbindung 2 sind vorzugsweise geschirmte Leitungen 3, 4, 5 vorhanden über welche die Tonausgabemittel 21, 22 sowie das Tonaufnahmemittel 23 mit einer AD/DA-Wandlereinheit 12 der Steuereinheit verbunden sind. Die AD/DA-Wandlereinheit 12 ist ihrerseits mit der Rechnereinheit 10 über wenigstens eine Leitung 6 für einen bi-direktionalen Datenaustausch verbunden. Alternativ zu der Kabelverbindung 2 könnte die Sonden-Einheit 20 auch drahtlos mit der Steuereinheit bzw. mit der Rechnereinheit 10 kommunizieren. Die drahtlose Verbindung könnte z. B. eine bluetooth-Funkstrecke oder eine andere geeignete Funkverbindung sein, die vorzugsweise eine kurze Reichweite aufweist.

Die Rechnereinheit 10 verfügt über einen Arbeitsspeicher 15 und über einen nicht-flüchtigen Speicher 16 in dem eine Modellfunktion $p_{dp,M} = f(L_1, L_2)$ für eine Modellpegelkarte eines menschlichen oder tierischen Gehörs und die zu dieser Modellfunktion gehörenden Parameter gespeichert sind. Ebenfalls ist in dem nicht-flüchtigen Speicher 16 die Anweisung zum Durchführen eines erfindungsgemäßen Verfahrens hinterlegt. Das System 1 verfügt ferner über ein Ausgabemittel 11 bzw. eine Anzeigeeinheit, wie z.B. ein Display, einen Monitor oder dergleichen, über die eine ermittelte einer individuellen Funktion einer DPOAE-Pegelkarte eines menschlichen oder tierischen Gehörs und deren Parameter von dem System 1 ausgegeben werden und einem Nutzer zugänglich gemacht werden können. Das Ausgabemittel 11 kann auch in Form einer Schnittstelle realisiert sein, über die ein externes Ausgabegerät, wie z.B. ein Drucker oder ein Monitor an das System angeschlossen werden können.

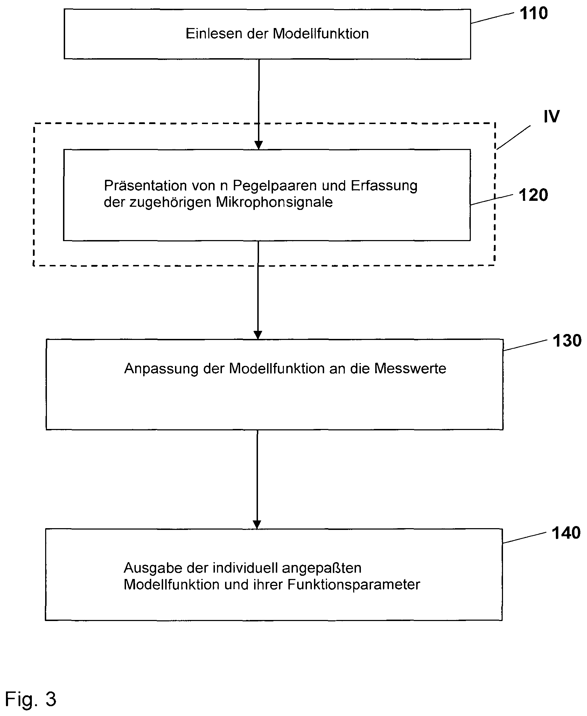

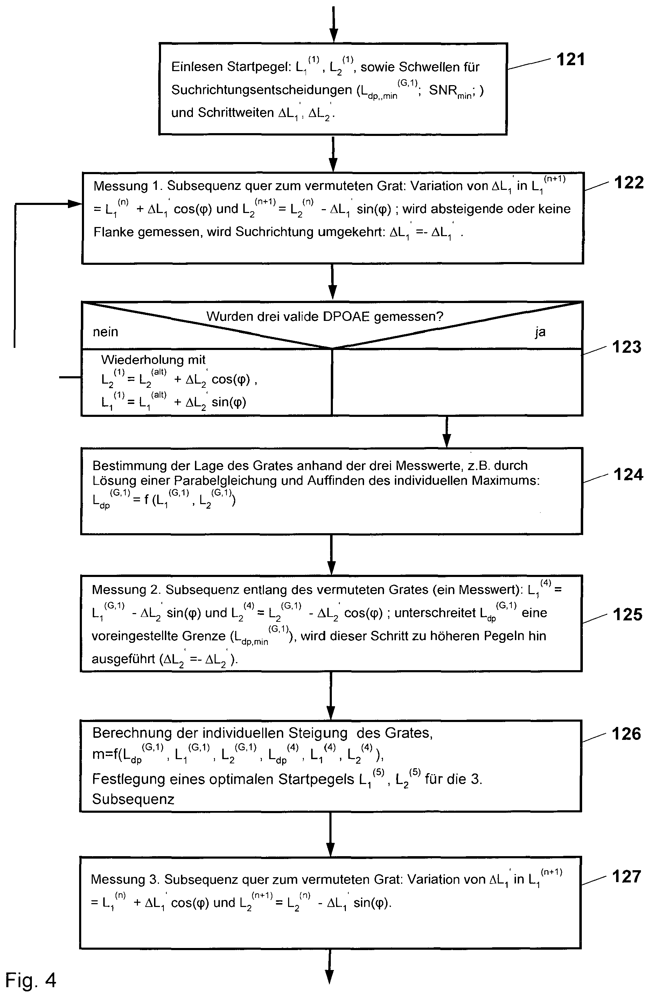

Zur Durchführung eines automatischen Messvorgangs zur Erstellung einer individuellen Funktion einer DPOAE-Pegelkarte eines menschlichen oder tierischen Gehörs wird die Sonden-Einheit 23 in Richtung des Pfeils 40 in den Gehörgang 31 eines Ohrs 30 (in Fig. 1 angedeutet) eingeführt. Das erfindungsgemäße Verfahren wird nachfolgend unter Bezugnahme auf die Figuren 3 und 4 erläutert.

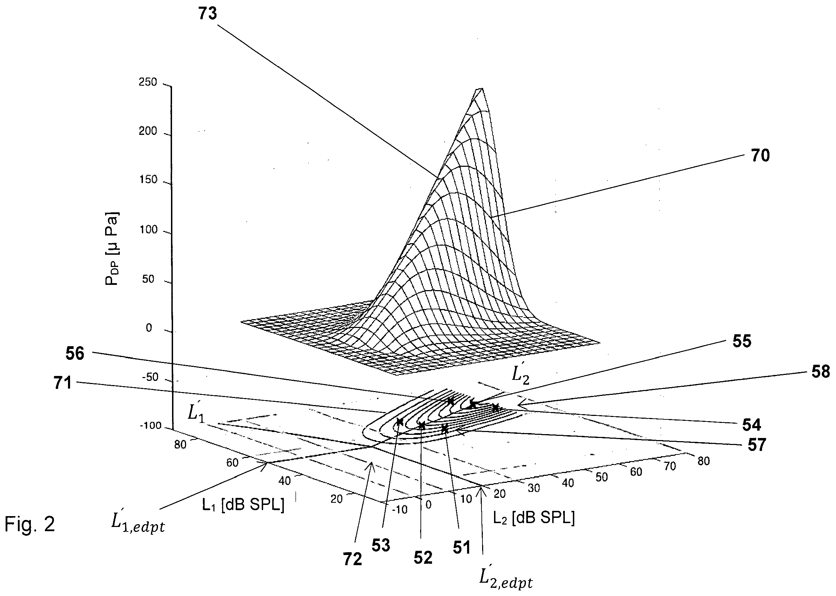

Zunächst ist aber in Figur 2 beispielhaft eine Modellfunktion dargestellt, deren dreidimensionaler Graph 70 einer Modellpegelkarte entspricht. Die beispielhaft dargestellte Modellfunktion beruht auf den Messdaten von $p \geq 2$, vorliegend p = 6, normalhörenden Individuen mit $N \geq 40$, vorliegend N= 47, gemessenen unterschiedlichen Pegelpaaren $\{L_1^{(1...N)}, L_2^{(1...N)}\}$ bei Anregungsfrequenzen f$_2$ = 2 kHz und f$_1$ = 1,67 kHz. Die Anregungsfrequenzen f$_2$ und f$_1$ eines Pegelpaares $\{L_1,L_2\}$ sind dabei vorzugsweise über ein Frequenzverhältnis f$_2$/f$_1$ = 1,2 verknüpft. Dabei wurde ein bestimmtes Distorsionsprodukt ausgewertet, welches bevorzugt bei der Frequenz f$_{dp}$ = 2f$_1$ - f$_2$ liegt. Dabei bedeuten die hochgestellten Indizes in den Klammern den 1. bis N. Meßpunkt.

Die Modellfunktion definiert einen näherungsweise linear ansteigenden Grat 73, dem näherungsweise linear

miteinander verknüpfte $\{L_1^{(G)}, L_2^{(G)}\}$ Pegelpaare zugeordnet sind. Linien quer zum Grat können durch die Beziehung $L_2$ + $aL_1$ = C definiert werden, wobei C eine beliebige Konstante, und wobei $a$ der Steigungsparameter der Projektion des Grates auf die $\{L_1, L_2\}$-Ebene ist. Im mathematischen Sinne ist die Lage des Grates durch eine hintereinanderhängende Schar von Gradientenvektoren des Skalarfeldes, das durch die DPOAE gebildet wird, definiert, wobei alle anderen durch Gradientenvektoren gebildeten Feldlinien auf diesen Grat zulaufen und einschwenken. In die unterhalb der Modellpegelkarte eingezeichnete, der Deutlichkeit halber um $p_{dp}$ = 100 μPa verschobene $\{L_1, L_2\}$-Ebene 71 ist das transformierte

$\{L_1', L_2'\}$-Koordinatensystem 72 eingezeichnet, welches durch Verschiebung des Ursprungs nach $\{L_{1,edpt}, L_{2,edpt}\}$ und Drehung um arctan(a) zustande kommt, sowie Höhenlinien der Pegelkarte im 20 μPa-Abstand. Die $L_2'$-Achse entspricht der Projektion des Grates der Pegelkarte auf die $\{L_1, L_2\}$-Ebene. Die $L_1'$-Achse schneidet den der Pegelkarte angenäherten Modell-Hügel orthogonal. Dieser Schnitt durch den Hügel quer zum Grat wird durch eine Parabel 2. Ordnung angenähert, deren Spreizung durch einen Parameter c gegeben ist, und der Eingang in folgende Gleichung findet:

$$L_{dp}' = -c(L_1')^2 + L_{dp}'^{(G)}$$

mit

$$L_{dp}'^{(G)} = 20\,log_{10}(m(L_2'))$$

$L_{dp}'$ und $L_{dp}'^{(G)}$ ist dabei der Pegel einer beliebigen bzw. einer auf dem Grat befindlichen DPOAE und m ist die Steigung des Grats entlang der $L_2'$-Achse.

Das $\{L_1', L_2'\}$ - Koordinatensystem befindet sich in der von dem bekannten Koordinatensystem $\{L_1, L_2\}$ der Primärtonpegel aufgespannten Fläche. Die vorhergehend bereits angesprochene Koordinatentransformation lässt sich z. B. wie folgt ausdrücken:

$$L_1' = \left(L_1 - L_{1,edpt}\right)\cos(\varphi) - \left(L_2 - L_{2,edpt}\right)\sin(\varphi)$$

$$L_2' = \left(L_1 - L_{1,edpt}\right)\sin(\varphi) + \left(L_2 - L_{2,edpt}\right)\cos(\varphi)$$

Dabei entspricht die Projektion des Grates des $L_{dp}$-Hügels auf die $\{L_1, L_2\}$-Ebene der $L_2'$ - Achse. Ferner entspricht der Punkt $\{L_{2,edpt}, L_{1,edpt}\}$ dem Fußpunkt des Grates des $L_{dp}$-Hügels, und φ ist der Winkel zwischen der $L_2$-Achse und der Projektion des Grates des $L_{dp}$-Hügels auf die $\{L_1, L_2\}$-Ebene, gegeben durch die bereits genannte $L_2'$-Achse. Der Winkel φp ist also der Winkel, um den die $L_2'$-Achse gegenüber der $L_2$-Achse gedreht ist. Der Fußpunkt des Grates kann in weiterem Sinne als äquivalent, aber nicht identisch zum "estimated distorsion product level" (edpt) interpretiert werden, wie er aus [P. Boege and T. Janssen., J. Acoust. Soc. Am., 111(4): 1810-1818, 2002] bekannt ist.

Die Modellfunktion für die Pegelkarte kann für den Gültigkeitsbereich positiver $L_{dp}$ durch fünf freie Parameter beschrieben werden: $a$; $b$; $c$; $L_{2,edpt}'$; $m$. Um aus Messwerten diese Fläche berechnen zu können, werden also mindestens 5 DPOAE benötigt.

Das erfindungsgemäße Verfahren beruht auf der Anpassung der dreidimensionalen Modellfunktion an eine grob abgetastete dreidimensionale DPOAE-Pegelkarte mit vorzugsweise wenigstens 5 Messungen. In einem ersten Ausführungsbeispiel des erfindungsgemäßen Verfahrens zur automatischen Bestimmung einer individuellen Funktion einer DPOAE-Pegelkarte mit $p_{dp}$ = $f(L_1, L_2)$ eines menschlichen oder tierischen Gehörs werden dem Gehör eines Individuums von dem aus Figur 1 ersichtlichen System n vordefinierte, z.B. n = 6 vordefinierte, Anregungspegelpaare $\{L_1, L_2\}$ 51, 52, 53, 54, 55, 56 präsentiert. Diese sechs vordefinierten Anregungspegelpaare $\{L_1, L_2\}$ 51, 52, 53, 54, 55, 56 sind beispielhaft in Figur 2 in der $\{L_1, L_2\}$-Ebene markiert. Aus diesen 6 vordefinierten Anregungspegelpaaren $\{L_1, L_2\}$ 51, 52, 53, 54, 55, 56, die in zwei Subsequenzen 57, 58 präsentiert werden, werden dann DPOAE ermittelt, die über das erfindungsgemäße Verfahren zur Ermittlung der individuellen Funktion einer DPOAE-Pegelkarte genutzt werden.

Gemäß Figur 3 wird in einem ersten Schritt 110 des erfindungsgemäßen Verfahrens zunächst die bereits beschriebene Modellfunktion aus dem nicht-flüchtigen Speicher 16 in die Rechnereinheit 10 des Systems 1, bzw. den Arbeitsspeicher 15 der Rechnereinheit 10, eingelesen. Nach dem Einlesen der Modellfunktion werden von dem System 1 im zweiten Schritt 120 eine Anzahl von unterschiedlichen Pegelpaaren $\{L_1^{(1...n)}, L_2^{(1...n)}\}$ eines Anregungsfrequenz-paars $\{f_1, f_2\}$ über die Tonausgabemittel 21, 22 der Sonden-Einheit 20 ausgegeben bzw. einem Individuum präsentiert und die korrespondierenden DPOAE des Individuums über die Tonaufnahmemittel 23 erfasst, wobei wenigstens das erste Pegelpaar $\{L_1^{(1)}, L_2^{(1)}\}$ vordefiniert ist und wobei $n \ll N$ ist. Die korrespondierenden DPOAE werden mittels der AD/DA-Wandlereinheit 12 der Rechnereinheit 10 zur Weiterverarbeitung zugeleitet. Dabei bedeuten die hochgestellten Indizes in den Klammern den 1. bis n. Meßpunkt.

Der zweite Schritt 120 beinhaltet in einer ersten Variante des erfindungsgemäßen Verfahrens, das als adaptive Variante bezeichnet werden kann, eine Reihe von Subschritten 121 bis 127, die nachfolgend mit Bezug auf Figur 4 näher erläutert werden.

Gemäß Figur 4 wird in einem ersten Subschritt 121 des zweiten Schritts 120 zunächst ein Startpegel $\{L_1^{(1)}, L_2^{(1)}\}$ (vorzugsweise ein Pegel $L_1$ von 67 ± 10 dB und ein Pegel $L_2$ von 57 ± 10 dB) für ein erstes Pegelpaar aus dem nicht-flüchtigen Speicher 16 in die Rechnereinheit 10 eingelesen. Ferner werden in diesem Subschritt 121 die Schrittweiten $\Delta L_1, \Delta L_2$ für die weiteren Pegelpaare $\{L_1^{(2...n)}, L_2^{(2..n)}\}$ sowie Schwellen für Suchrichtungsentscheidungen $L_{dp,min}^{(G,1)}$ ; $SNR_{min}$) in die Rechnereinheit 10 eingelesen. $SNR_{min}$ bezeichnet dabei das ge-wünschte SNR (signal-to-noise ratio - Rauschabstand) und $L_{dp,min}^{(G,1)}$ bezeichnet dabei den DPOAE-Pegel auf dem Grat, der mindestens entlang einer ersten Subsequenz von k Subsequenzen vorliegen muß, damit eine nächste Subsequenz unterhalb der ersten noch mit ausreichendem SNR erwartet werden kann. Wird dieser Wert nicht erreicht, dann wird die nächste Subsequenz oberhalb der ersten, also grataufwärts, abgetastet, um eine zu lange Messzeit zum Erreichen eines ausreichenden SNR zu vermeiden. Die Schrittweiten $\Delta L_1, \Delta L_2$ bezeichnen den Pegelabstand von zwei aufeinanderfolgenden Pegelpaaren wobei $\Delta L_1$ insbesondere einen Wert von $\Delta L_1$ = 4 bis 14 dB, vorzugsweise von 6 bis 10 dB aufweist und wobei $\Delta L_2$ = 0 bis -2,78 dB ist, vorzugsweise $\Delta L_2$ = - 1,52 bis -2,78 dB ist.

In einem zweiten Subschritt 122 des zweiten Schritts erfolgen nun die Messungen der ersten Subsequenz von $k$ Subsequenzen quer zum vermuteten Grad der individuellen Funktion einer Pegelkarte. Die DPOAE werden dabei bei den bereits oben beschriebenen Anregungsfrequenzen $f_2$ = 2 kHz und $f_1$ = 1,67 kHz durchgeführt. Die Anregungsfrequenzen $f_2$ und f, eines Pegelpaares $\{L_1, L_2\}$ sind dabei vorzugsweise über ein Frequenzverhältnis von etwa $f_2/f_1$ = 1,2 verknüpft. Die Subsequenz gehört dabei zu einer Anzahl von k Subsequenzen, wobei $k \geq 2$ und $\leq 5$ ist. In jeder Subsequenz werden dabei eine Anzahl von $n_k$ Pegelpaaren $\{L_1, L_2\}$ gemessen.

Der Startpegel $\{L_1^{(1)}, L_2^{(1)}\}$ wird entsprechend den festgelegten Schrittweiten $\Delta L_1, \Delta L_2$ variiert entsprechend der Formel $L_1^{(n+1)} = L_1^{(n)} + \Delta L_1' \cos(\varphi)$ und der weiteren Formel $L_2^{(n+1)} = L_2^{(n)} - \Delta L_1' \sin(\varphi)$ . Wird in die gemessene Subsequenz eine absteigende oder keine ansteigende Flanke gemessen, wird die Suchrichtung umgekehrt und $\Delta L_1' = - \Delta L_1'$

In einem dritten Subschritt 123 wird überprüft, ob wenigstens drei valide DPOAE gemessen wurden. Ist diese Überprüfung positiv, das heißt es wurden drei valide DPOAE gemessen, geht das Verfahren weiter zum nächsten Subschritt 124. Wurden keine drei validen DPOAE gemessen, dann erfolgt eine Wiederholung der Messung, bei der aus dem ursprünglichen Anregungspegel L_1^(alt) , L_2^(alt) ein neuer Anregungspegel L_1^(1), L_2^(1) nach

$$L_2^{(1)} = L_2^{(alt)} + \Delta L_2' \cos(\varphi) ,$$

$$L_1^{(1)} = L_1^{(alt)} + \Delta L_2' \sin(\varphi)$$

bestimmt wird.

In dem nachfolgenden vierten Subschritt 124 erfolgt die Bestimmung der Lage des Grates der individuellen Funktion anhand der drei valide bestimmten Messwerte, z.B. durch Lösung einer Parabelgleichung und Auffinden des individuellen Maximums nach der Funktion:

$$L'^{(G,1)}_{dp} = f(L_1^{(1..3)}, L_2^{(1..3)})$$

Hier bedeutet $L'^{(G,1)}_{dp}$ denjenigen Punkt auf dem geschätzten Grat der individuellen Modellfunktion, dessen zugehöriges Anregungspegelpaar auf der durch $\{L_1^{(1..3)}, L_2^{(1..3)}\}$ gebildeten Linie liegen. Die Lage des Grates der individuellen Funktion bei den höheren Anregungspegeln $L^{(1..3)}$ ist nun also bereits aus dem Subschritt 124 bekannt, die Steigung des Grates, d.h. der Parameter m, jedoch nicht.

Im nachfolgenden fünften Subschritt 125 des zweiten Schritts 120 erfolgt eine Messung einer zweiten Subsequenz entlang des vermuteten Grates, wobei lediglich ein Messwert ermittelt wird. Die Messung erfolgt nach der Formel

$$L_1^{(4)} = L'^{(G,1)}_{dp} - \Delta L'_2 \sin(\varphi) .$$

Unterschreitet $L'^{(G,1)}_{dp}$ eine voreingestellte Grenze ( $L'^{(G,1)}_{dp,min}$ ), wird dieser Schritt zu höheren Pegeln hin ausgeführt ($\Delta L_2 = - \Delta L_2$).

Der dort gemessene Wert des DPOAE ( $L_{dp}^{(4)}$ ) wird nachfolgend im sechsten Subschritt 126 dazu verwendet, die individuelle Steigung des Grates, m, zu bestimmen.

In dem sechsten Subschritt 126 des zweiten Schritts 120 erfolgt die Berechnung der individuellen Steigung des Grates nach der Formel $m=f(L_{dp}^{(G,1)}, L_1^{(G,1)}, L_2^{(G,1)}, G_{dp}^{(4)}, L_1^{(4)}, L_2^{(4)})$. Anhand der ermittelten Steigung m des Grates erfolgt nun die Festlegung eines Startpegels $L_1^{(5)}, L_2^{(5)}$ für die dritte Subsequenz.

Im siebten Subschritt 127 des zweiten Schritts 120 erfolgen nun die Messungen der dritten Subsequenz quer zu dem vermuteten Grat der Funktion: Es erfolgt eine Variation von $\Delta L'_1$ in $L_1^{(n+1)} = L_1^{(n)} + \Delta L'_1 \cos(\varphi) .$

Vorzugsweise liegen mindestens die Hälfte der Pegelpaare $\{L_1, L_2\}$, bei denen gemessen wird, um mindestens 5 dB zu beiden Seiten abseits der Gruppe der dem Grat (der Modellfunktion) zugeordneten $\{L_1^{(G)}, L_2^{(G)}\}$ Pegelpaare.

Mit den gemäß dem zweiten Schritt 120 und seinen Subschritten 121 bis 127 ermittelten Messwerten, erfolgt nun eine Anpassung der bereits vorgestellten Modellfunktion an die gewonnenen Messwerte in einem dritten Schritt 130 in der Rechnereinheit 10. Hierbei wird an die gemessenen DPOAE-Werte die dreidimensionale Modellfunktion $p_{dp,M} = f(L_1, L_2)$ angepaßt. Die Anpassung erfolgt mit den mathematischen Methoden der Ausgleichsrechnung, z.B. mit der Methode der kleinsten Quadrate, d.h., mit der iterativen Minimierung der Differenz zwischen den n Messwerten und den Werten der Modellfunktion $p_{dp,M} = f(L_1, L_2)$ an die gemessenen n DPOAE (enstprechend den zugehörigen $L_1$, $L_2$-Koordinaten) bis zum Erhalt einer individuellen Funktion $p_{dp,I} = f(L_1, L_2)$ mit individuellen Parametern einer DPOAE-Funktion und -Pegelkarte des Individuums durch die Rechnereinheit 10. Man erhält damit auf einfache Weise eine individuelle Funktion / Pegelkarte des Gehörs eines Individuums mit stark verringertem messtechnischem Aufwand in kurzer Zeit.

In einem vierten Schritt 140 erfolgt nun die Ausgabe der individuell angepassten Funktion und ihrer Funktionsparameter in einem Ausgabemittel 11 des Systems 1, wie z.B. einem Display, Monitor, Drucker, etc.. Die ausgegebenen Funktionsparameter beinhalten dabei insbesondere die bereits oben beschriebenen Parameter *a; b; c;* $L'_{2,edpt}$ ; und die Steigung des Grates m. Das Ausgabemittel 11 kann, wie bereits erwähnt, auch in Form einer Schnittstelle realisiert sein, über die ein externes Ausgabegerät, wie z.B. ein Drucker oder ein Monitor an das System 1 angeschlossen werden können.

In einem möglichen weiteren Verfahrensschritt, kann die ermittelte individuelle Funktion einer DPOAE-Pegelkarte und deren Parameter von der Rechnereinheit 10 in dem nicht-flüchtiger Speicher 16 abgespeichert werden. Auch können die gemessenen Rohdaten von der Rechnereinheit 10 in dem nicht-flüchtiger Speicher 16 abgespeichert werden. Die abgespeicherten Daten können von der Rechnereinheit 10 z.B. zur kontinuierlichen Erweiterung des, der Modellfunktion einer Pegelkarte zugrundeliegenden Datensatzes verwendet werden.

Aus der erfindungsgemäß erhaltenen individuell angepassten Funktion und den dazugehörigen Funktionsparametern können folgende Erkenntnisse gewonnen werden:

- Es kann eine angenäherte Distorsionsproduktschwelle berechnet werden, die Auskunft über die Schwelle des Eingangssignals für die inneren Haarzellen des gemessenen Gehörs liefert. Als entsprechenden Parameter kann $L_{2,edpt}$ angesehen werden.

- Die Breite des Grates, in der Funktion durch den Parameter c beschrieben, ist ein Maß für die Kompression und damit für die Frequenzauflösung der zugrundeliegenden Wanderwellen im gemessenen Gehör.

- Die Lage und der Winkel, in der Funktion ausgedrückt durch die Parameter $a$; $b$ enthält Informationen über die Natur eines Hörverlustes: Bei einem reinen Schallleitungsverlust verändert sich der Winkel (in der Funktion ausgedrückt durch den Parameter a) nicht, stattdessen verschiebt sich der Hügel in erster Näherung in gleichem Maße in Richtung höherer $L_1$- und $L_2$-Pegel. Wenn sich z.B. die Verschiebung des Hügels (gegenüber den Normwerten, oder gegenüber einer Referenzmessung des Individuums zu einem früheren Zeitpunkt) mit der Verschlechterung der Distorsionsproduktschwelle deckt, also $\Delta L_2 \approx \Delta L_1 \approx \Delta L_{2,edpt}$ kann auf einen reinen Schallleitungsverlust geschlossen werden.

- Die Steilheit des Grates, ausgedrückt durch den Parameter m, lässt Rückschlüsse auf einen möglichen Schallleitungsverlust zu. Solange der Hörverlust unterhalb 30 dB liegt, kann erwartet werden, daß bei einem reinen Schallleitungsverlust die Steigung den Normwerten entspricht, während bei einer Abweichung gegenüber dem Normwert eine proportionale Reduzierung der retrograden Mittelohrübertragung bei $f_{dp}$ indiziert ist.

In einer alternativen Variante des erfindungsgemäßen Verfahrens wird anstelle der Sub-Schritte 121 bis 127 im Rahmen der Messungen des zweiten Schritts 120 eine Anzahl von $n$ festgelegten bzw. vordefinierten aber unterschiedlichen Pegelpaaren $\{L_1,L_2\}$ (wobei n vorzugsweise $\geq 5$ und $\leq 12$ ist, insbesondere $\geq 5$ und $\leq 8$) vom System ausgegeben und die Reaktion des Gehörs eines Individuums auf diese Pegelpaare $\{L_1,L_2\}$ erfasst. Diese Variante kann dabei als starres Verfahren bezeichnet werden. Die Pegelpaare $\{L_1,L_2\}$ können dabei wiederum in einer Anzahl $k$ Subsequenzen (57, 58; vgl. Fig. 2) gemessen werden, wobei $k \geq 2$ und $\leq 12$ ist. Die n Pegelpaare sind dabei dann weitestgehend statisch und es erfolgt keine Anpassung der zweiten und ggf. dritten Subsequenz auf die Ergebnisse der Messungen der ersten Subsequenz, wie es bei dem vorhergehend geschilderten Verfahren der Fall ist. Die Anzahl n der Pegelpaare $\{L_1,L_2\}$ ist dabei nach einer Abwägung von Meßzeit (möglichst wenig Meßpunkte) gegenüber erzielbarer Genauigkeit (möglichst viele Punkte) ausgelegt.

In diesem starren Verfahren mit fest vorgegebenen Anregungspegeln kann bspw. mit $L_2' = 40\,\mathrm{dB}$ für die drei höheren Anregungspegel, und $L_2' = 25\,\mathrm{dB}$ für die drei niedrigeren Anregungspegel, und innerhalb einer Gruppe von drei Anregungspegeln jeweils $L_1' = 0 \pm 6\,\mathrm{dB}$ gemessen werden. Im $\{L_1,L_2\}$-Koordinatensystem entspricht das den Anregungspegeln $L_2 = 68,1; 65,6; 63,1; 42,8; 45,3; 40,3$ und $L_1 = 68,0; 73,5; 79,0; 63,3; 57,8; 68,7$ (vgl. hierzu Fig. 2). Dabei verfolgt die Wahl $L_1' = 0 \pm 6\,\mathrm{dB}$ das Ziel, mit drei Punkten, die quer zur vermuteten Lage des Grates liegen, dessen Lage sicher einzumessen: bei $f_2$ = 2 kHz fällt das DPOAE mit $\Delta L_1' \pm 6\,\mathrm{dB}$ typischerweise auf etwa 50% des Maximalwertes ab. Wenn in erster Linie normalhörende Individuen gemessen werden sollen, wie es üblicherweise bei Siebtests der Fall ist, wird die starre Anordnung gute Ergebnisse liefern. Ausreißer müssen aber erkannt werden. Dies ist über den quadratischen Fehler der Modellanpassung möglich. Ist der Fehler zu hoch, d.h. ist der rms-Fehler (rms: rootmean-square) beispielsweise größer als $5\ \mu Pa,$ müssen weitere Pegelpaare gemessen werden, bis der Fehler gering genug ist. Hier bieten sich weitere $\Delta L_1'$ - Schritte an. Ebenso muß verfahren werden, wenn wegen zu geringem Rauschabstand einzelne Meßpunkte nicht registriert werden können.

Abschließend bleibt noch anzumerken, dass abweichend von dem verwendeten und vorgängig beschriebenen Frequenzverhältnis $f_2/f_1$ von 1,2 auch ein anderes Frequenzverhältnis gewählt werden kann. So kann das Frequenzverhältnis $f_2/f_1$ z. B. auch auf einen anderen geeigneten Wert zwischen 1,15 und 1,35 festgelegt werden. Ferner könnte das Frequenzverhältnis $f_2/f_1$ eine Funktion von f2 sein.

<u>Bezugszeichenliste</u>

1    System
2    Kabelverbindung
3    erste Leitung
4    zweite Leitung
5    dritte Leitung
6    vierte Leitung

10    Rechnereinheit
11    Ausgabemittel
12    AD/DA-Wandlereinheit
13    DA-Wandler

14    AD-Wandler
15    Arbeitsspeicher
16    nicht-flüchtiger Speicher mit gespeicherter Modellfunktion

20    Sonden-Einheit, OAE-Sonde
21    erstes Tonausgabemittel, f1-Schallgeber
22    zweites Tonausgabemittel, f2-Schallgeber
23    Tonaufnahmemittel, Mikrophon
24    Sondenspitze

30    Ohr
31    Gehörgang

40    Pfeil

51    Anregungspegelpaar $\{L_1, L_2\}$
52    Anregungspegelpaar $\{L_1, L_2\}$
53    Anregungspegelpaar $\{L_1, L_2\}$
54    Anregungspegelpaar $\{L_1, L_2\}$
55    Anregungspegelpaar $\{L_1, L_2\}$
56    Anregungspegelpaar $\{L_1, L_2\}$
57    erste Subsequenz
58    zweite/weitere Subsequenz

70    Graph / Modellpegelkarte
71    $\{L_1, L_2\}$-Ebene
72    transformiertes $\{L_1', L_2'\}$ -Koordinatensystem
73    Grat (der DPOAE Modellpegelkarte)

110    erster Verfahrensschritt
120    zweiter Verfahrensschritt
121    erster Subschritt
122    zweiter Subschritt
123    dritter Subschritt
124    vierter Subschritt
125    fünfter Subschritt
126    sechster Subschritt
127    siebter Subschritt
130    dritter Verfahrensschritt
140    vierter Verfahrensschritt

**Patentansprüche**

1. Verfahren, zur automatischen Bestimmung einer individuellen Funktion einer DPOAE-Pegelkarte mit $p_{dp,I}$ = $f(L_1, L_2)$ eines menschlichen oder tierischen Gehörs, bei der Distorsionsprodukt-otoakustische Emissionen (DPOAE) als Schalldruck $p$ in Abhängigkeit von den Pegeln $L_1$ und $L_2$ der zur Erzeugung der DPOAE verwendeten zwei Primärtöne dargestellt werden, **dadurch gekennzeichnet, dass** es die folgenden Schritte beinhaltet:

- Einlesen (110) einer Modellfunktion (70) $p_{dp,M}$ = $f(L_1, L_2)$ mit Modellparametern einer DPOAE-Pegelkarte, basierend auf einer Anzahl von N DPOAE-Messungen eines Anregungsfrequenzpaars $f_1$, $f_2$ mit jeweils unterschiedlichen Pegelpaaren $\{L_1^{(1...N)}, L_2^{(1...N)}\}$ bei einer Population *(p)* von Normalhörenden, in einen Arbeitsspeicher (15) einer Rechnereinheit (10), wobei $N \geq 40$ und $p \geq 2$ ist,

- automatische Präsentation (120) von *n* unterschiedlichen Pegelpaaren $\{L_1^{(1...n)}, L_2^{(1...n)}\}$ eines Anregungsfrequenzpaars $f_1$, $f_2$ über Tonausgabemittel (21, 22) an ein Individuum und Erfassen der korrespondierenden DPOAE des Individuums über Tonaufnahmemittel (23), wobei wenigstens das erste Pegelpaar

$\{L_1^{(1)}, L_2^{(1)}\}$ vordefiniert ist und wobei n « N ist,

- iteratives Anpassen (130) der Modellfunktion $p_{dp,M} = f(L_1, L_2)$ an die gemessenen n DPOAE bis zum Erhalt einer individuellen Funktion $p_{dp,I} = f(L_1, L_2)$ mit individuellen Parametern einer DPOAE-Pegelkarte des Individuums durch die Rechnereinheit (10),

- Ausgabe (140) der individuellen Funktion $p_{dp,I} = f(L_1, L_2)$ und/oder deren individueller Parameter an einem Ausgabemittel (11) der Rechnereinheit (10), wobei das Verfahren **dadurch gekennzeichnet ist, dass** die Modellfunktion einen näherungsweise linear ansteigenden Grat (73) aufweist, dem näherungsweise linear miteinander verknüpfte $\{L_1^{(G)}, L_2^{(G)}\}$ Pegelpaare zugeordnet sind, wobei mindestens die Hälfte der gemessenen Pegelpaare $\{L_1, L_2\}$, um mindestens 5 dB zu beiden Seiten abseits der Gruppe der dem Grat (73) zugeordneten $\{L_1^{(G)}, L_2^{(G)}\}$ Pegelpaare liegen.

2. Verfahren nach Anspruch 1, **dadurch gekennzeichnet, dass** das erste Pegelpaar $\{L_1^{(1)}, L_2^{(1)}\}$ einen Pegel $L_1^{(1)}$ von 67 $\pm$ 10 dB und einen Pegel $L_2^{(1)}$ von 57 $\pm$ 10 dB aufweist.

3. Verfahren nach einem der vorhergehenden Ansprüche, **dadurch gekennzeichnet, dass** die unterschiedlichen Pegelpaare $\{L_1, L_2\}$ in einer Sequenz präsentiert werden die für jedes Individuum identisch ist.

4. Verfahren nach einem der vorhergehenden Ansprüche, **dadurch gekennzeichnet, dass** die vordefinierten, unterschiedlichen Pegelpaare $\{L_1, L_2\}$ in einer Sequenz präsentiert werden, die eine Anzahl von $k$ Subsequenzen aufweisen , deren Pegelpaare $\{L_1, L_2\}$ im Wesentlichen quer zu den linear miteinander verknüpften und dem Grat zugeordneten Pegelpaaren $\{L_1^{(G)}, L_2^{(G)}\}$ liegen.

5. Verfahren nach einem der vorhergehenden Ansprüche, **dadurch gekennzeichnet, dass** $n \geq 5$ und $\leq 12$ ist, vorzugsweise $n \geq 5$ und $\leq 8$ ist.

6. Verfahren nach einem der vorhergehenden Ansprüche, **dadurch gekennzeichnet, dass** $k \geq 2$ und $\leq 8$.

7. Verfahren nach einem der vorhergehenden Ansprüche, **dadurch gekennzeichnet, dass** die einem ersten vordefinierten Pegelpaar $\{L_1^{(1)}, L_2^{(1)}\}$ nachfolgenden Pegelpaare $\{L_1^{(2..n_k)}, L_2^{(2..n_k)}\}$ einer Subsequenz mit $n_k$ Messungen über eine Funktion $\{L_1^{(i)}, L_2^{(i)}\} = \{L_1^{(i-1)} + \mu \cdot \Delta L_1, L_2^{(i-1)} + \mu \cdot \Delta L_2\}$ aus dem jeweils vorhergehenden Pegelpaar $\{L_1^{(i-1)}, L_2^{(i-1)}\}$ bestimmt werden, wobei $\mu = \pm 1$, vorzugsweise +1 ist, und $\Delta L_1$, $\Delta L_2$ ein Pegelabstand von zwei aufeinanderfolgenden Pegelpaaren ist und Werte von $\Delta L_1 = 4$ bis 14 dB, vorzugsweise von 6 bis 10 dB, und $\Delta L_2 = 0$ bis - 2,78 dB, vorzugsweise $\Delta L_2 = -1,52$ bis - 2,78 dB aufweist.

8. Verfahren nach einem der vorhergehenden Ansprüche, **dadurch gekennzeichnet, dass**, wenn das erste Pegelpaar $\{L_1^{(1)}, L_2^{(1)}\}$ und das zweite Pegelpaar $\{L_1^{(2)}, L_2^{(2)}\}$ zwei DPOAE mit $p_{dp,I}^{(12)}$ produziert, die einen Rauschabstand von je >= 4 *dB*, vorzugsweise >= *10 dB* aufweisen, der Pegel eines nachfolgendes dritten Pegelpaars $\{L_1^{(3)}, L_2^{(3)}\}$ wenigstens um $\Delta L_1 \geq 4$ dB unterschiedlich eingestellt wird als der Pegel des vorhergehenden Pegelpaars $\{L_1^{(2)}, L_2^{(2)}\}$, wenn $p_{dp,I}^{(2)} - p_{dp,I}^{(1)} > 0$, andererseits der Pegel eines nachfolgendes Pegelpaars $\{L_1^{(3)}, L_2^{(3)}\}$ wenigstens um $\Delta L_1 \leq -4$ dB unterschiedlich eingestellt wird als der Pegel des ersten Pegelpaars $\{L_1^{(1)}, L_2^{(1)}\}$, wenn $p_{dp,I}^{(2)} - p_{dp,I}^{(1)} \leq 0$.

9. Verfahren nach einem der vorhergehenden Ansprüche, **dadurch gekennzeichnet, dass**, wenn das erste Pegelpaar $\{L_1^{(1)}, L_2^{(1)}\}$ keine DPOAE mit $p_{dp,I}^{(1)}$ produziert, die einen Rauschabstand von $\geq 4$ *dB*, vorzugsweise $\geq 10$ *dB*

aufweist, in derselben Suchrichtung weiterverfahren wird, bis entweder der maximale bzw. minimale Anregungspegel $L_1^{(i)}$ erreicht ist, oder eine Gruppe von drei validen DPOAE mit $p_{dp,l}{}^{(i..i+2)}$ produziert wurde, die einen Rauschabstand von je $\geq 4$ *dB*, vorzugsweise $\geq 10$ *dB* aufweisen.

10. Verfahren nach einem der vorhergehenden Ansprüche, **dadurch gekennzeichnet, dass**, wenn in der ersten Subsequenz keine Gruppe von drei validen DPOAE produziert wird, die einen Rauschabstand von je $\geq 4$ *dB*, vorzugsweise $\geq 10$ *dB* aufweisen, eine erneute Subsequenz mit höherem Pegelpaar $\{L_1^{(i+1)}, L_2^{(i+1)}\}$, gestartet wird, wobei das Startpegelpaar für die erneute Subsequenz auf $L_2^{(i+3)} = L_2^{(1)} + 20 \pm 10\,dB$, $L_1^{(i+3)} = L_1^{(1)} + 20 \pm 10\,dB$, höchstens aber auf die maximal erreichbaren Pegel eingestellt wird.

11. Verfahren nach einem der vorhergehenden Ansprüche, **dadurch gekennzeichnet, dass** nach der Erfassung der DPOAE von wenigstens 3 Pegelpaaren $\{L_1^{(1..3)}, L_2^{(1..3)}\}$, vor- zugsweise einer Subsequenz, aus diesen 3 Pegelpaaren $\{L_1^{(1..3)}, L_2^{(1..3)}\}$ die Lage des Grates $\{L_1^{(G)}, L_2^{(G)}\}$ längs der durch die 3 Pegelpaare gebildeten Linie ermittelt wird und ein viertes Pegelpaar $\{L_1^{(4)}, L_2^{(4)}\}$ präsentiert wird, welches in einem vorgegebenen Abstand gratabwärts gelegt wird, wobei der Gruppenmittelwert der Gratrichtung, $\varphi$ verwendet wird, und wobei anhand der aus den vier präsentierten Pegelpaaren $\{L_1^{(1..4)}, L_2^{(1..4)}\}$ bestimmten DPOAE eine Steigung des linearen Grats der Pegelkarte ermittelt wird.

12. Verfahren nach Anspruch 1 oder 11, **dadurch gekennzeichnet, dass**, wenn in der ersten oder zweiten Subsequenz eine Gruppe von drei validen DPOAE mit $p_{dp,l}{}^{i-2...i}$ produziert wird, die einen Rauschabstand von je >=4 dB, vorzugsweise >=10 dB aufweisen, durch Anpassung einer mathematischen Funktion an die zugehörigen DPOAE $p_{dp,l}{}^{i-2...i}$ das Pegelpaar unterhalb des Grates $\{L_1^{(G)}, L_2^{(G)}\} = \{L_1^{(i-2)} + \varepsilon \cdot \Delta L_1, L_2^{(i-2)} + \varepsilon \cdot \Delta L_2\}$ bestimmt wird, wobei $\varepsilon$ so berechnet werden muß, daß $p_{dp,l}(L_1^{(g)}, L_2^{(g)})$ ein Maximum bildet, und davon ausgehend ein viertes Pegelpaar $L_1^{(i+1)}, L_2^{(i+1)}$ präsentiert wird, mit einer Funktion $\{L_1^{(i+1)}, L_2^{(i+1)}\} = \{L_1^{(i)} + \Delta L_1, L_2^{(i)} + \Delta L_2\}$, wobei $\Delta L_2 = -15 \pm 10$ *dB* eingestellt wird, und das Pegelpaar vorzugsweise auf der Projektion des erwarteten Grates auf die $L_1, L_2$-Ebene, d.h. mit $\Delta L_1 / \Delta L_2 \approx 0.51 \pm 0.15$ eingestellt wird, und wobei anhand der aus den vier präsentierten Pegelpaaren $L_1^{(i-2...i+1)}, L_2^{(i-2...i+1)}$ bestimmten DPOAE eine Steigung m des näherungsweise linearen Grats der Pegelkarte ermittelt wird.

13. Verfahren nach einem der vorhergehenden Ansprüche, **dadurch gekennzeichnet, dass** die Pegelpaare $\{L_1^{(1..n)}, L_2^{(1..n)}\}$ gepulst präsentiert werden, wobei jeder einzelne Puls mit einer Dauer $T_D$ von 2 bis 40 ms präsentiert wird.

14. Verfahren nach Anspruch 13, **dadurch gekennzeichnet, dass** die Pegelpaare $\{L_1^{(1..n)}, L_2^{(1..n)}\}$ innerhalb eines Messblocks aus mehreren, zeitlich nacheinander in Pulsen präsentierten Pegelpaaren $\{L_1^{(1..n,m)}, L_2^{(1..n,m)}\}$ präsentiert werden, wobei zeitlich direkt aufeinanderfolgende Pegelpaare $\{L_1^{(1..n,i)}, L_2^{(1..n,i)}\}; \{L_1^{(1..n,i+1)}, L_2^{(1..n,i+1)}\}$ unterschiedliche Anregungsfrequenzen $\{f_{2,i}, f_{1,i}\}; \{f_{2,i+1}, f_{1,i+1}\}$ aufweisen, wobei insbesondere in einem weiteren Verfahrensschritt, die ermittelte individuelle Funktion einer DPOAE-Pegelkarte und deren Parameter von der Rechnereinheit (10) in einem nicht-flüchtigen Speicher (16) abgespeichert wird.

15. System eingerichtet zur Durchführung des Verfahrens nach einem der Ansprüche 1 bis 14, mit einer Rechnereinheit 10, mit einem Arbeitsspeicher (15), mit einem nicht-flüchtigen

Speicher (16) zur Speicherung einer Modellfunktion $p_{dp\,M} = f(L_1, L_2)$ und Modellparametern der Modellfunktion, mit wenigstens einem über die Rechnereinheit (10) angesteuerten Tonausgabemittel (21, 22) zur Präsentation von Tönen an ein Individuum, mit wenigstens einem mit der Rechnereinheit (10) verbundenen Tonaufnahmemittel (23) zum Erfassen von DPOAE aus dem Ohr des Individuums, wobei insbesondere das wenigstens eine Tonausgabemittel (21, 22) ein Lautsprecher mit einer hochlinearen Charakteristik ist und/oder wobei insbesondere ein Ausgabemittel (11) zur Ausgabe der individuellen Funktion einer DPOAE-Pegelkarte an einen Nutzer vorgesehen ist.

**Claims**

1. Method for the automated determination of an individual function of a DPOAE level map with $p_{dp,I} = f(L_1, L_2)$ of human or animal hearing, wherein distortion product otoacoustic emissions (DPOAEs) are presented as acoustic pressure p in dependence on levels $L_1$ and $L_2$ of the two primary tones used for the generation of DPOAEs, **characterized in that** it comprises the following steps:

   - reading (110) of a model function (70) $p_{dp,M} = f(L_1, L_2)$ with model parameters of a DPOAE level map, based upon a number of N DPOAE measurements of a stimulation frequency pair $f_1, f_2$ with respectively different level pairs $\{L_1^{(1...N)}, L_2^{(1...N)}\}$ in a population (p) of normally hearing subjects into a main memory (15) of a computer unit (10), wherein $N \geq 40$ and $p \geq 2$,
   - automatically presenting (120) n different level pairs $\{L_1^{(1...n)}, L_2^{(1...n)}\}$ of a stimulation frequency pair $f_1, f_2$ via tone output means (21, 22) to an individual and detecting the corresponding DPOAEs of the individual via tone recording means (23), wherein at least the first level pair $\{L_1^{(1)}, L_2^{(1)}\}$ is predefined, and wherein $n << N$,
   - iteratively adapting (130) the model function $p_{dp,M} = f(L_1, L_2)$ to the measured n DPOAEs until an individual function $p_{dp,I} = f(L_1, L_2)$ is obtained with individual parameters of a DPOAE level map of the individual by the computer unit (10), and
   - outputting (140) the individual function $p_{dp,I} = f(L_1, L_2)$ and/or its individual parameters to an output means (11) of the computer unit (10),

   wherein the method is **characterized in that** the model function has a more or less linearly rising ridge (73) to which more or less linearly linked level pairs $\{L_1^{(G)}, L_2^{(G)}\}$ are assigned, wherein at least half of the measured level pairs $\{L_1, L_2\}$ lie at least 5 dB remote from either side of the group of the level pairs $\{L_1^{(G)}, L_2^{(G)}\}$ assigned to the ridge (73).

2. Method according to claim 1, **characterized in that** the first level pair $\{L_1^{(1)}, L_2^{(1)}\}$ has a level $L_1^{(1)}$ of $67 \pm 10$ dB and a level $L_2^{(1)}$ of $57 \pm 10$ dB.

3. Method according to any one of the above claims, **characterized in that** the different level pairs $\{L_1, L_2\}$ are presented in a sequence that is identical for each individual.

4. Method according to any one of the above claims, **characterized in that** the predefined, different level pairs $\{L_1, L_2\}$ are presented in a sequence that has a number of k subsequences whose level pairs $\{L_1, L_2\}$ are essentially transverse to the linearly linked level pairs $\{L_1^{(G)}, L_2^{(G)}\}$ assigned to the ridge.

5. Method according to any one of the above claims, **characterized in that** n is $\geq 5$ and $\leq 12$, and preferably n is $\geq 5$ and $\leq 8$.

6. Method according to any one of the above claims, **characterized in that** $k \geq 2$ and $\leq 8$.

7. Method according to any one of the above claims, **characterized in that** the level pairs $\{f_1^{(2...nk)}, L_2^{(2..nk)}\}$ of a subsequence following a first predefined level pair $\{L_1^{(1)}, L_2^{(1)}\}$ is determined with $n_k$ measurements by a function $\{L_1^{(i)}, L_2^{(i)}\} = \{L_1^{(i-1)} + \mu \cdot \Delta L_1, L_2^{(i-1)} + \mu \cdot \Delta L_2\}$ from the respectively preceding level pair $\{L_1^{(i-1)}, L_2^{(i-1)}\}$, wherein $\mu = \pm 1$, preferably +1, and $\Delta L_1, \Delta L_2$ is a level distance of two sequential level pairs and has values of $\Delta L_1 = 4$ to 14 dB, preferably from 6 to 10 dB - and $\Delta L_2 = 0$ to -2.78 dB , preferably $\Delta L_2 = -1.52$ to -2.78 dB.

8. Method according to any one of the above claims, **characterized in that**, when the first level pair $\{L_1^{(1)}, L_2^{(1)}\}$ and the second level pair $\{L_1^{(2)}, L_2^{(2)}\}$ produce two DPOAEs with $p_{dp,I}^{(12)}$ that each have a signal-to-noise ratio of >= *4 dB,* preferably >= 10 dB, the level of a subsequent third level pair $\{L_1^{(3)}, L_2^{(3)}\}$ is adjusted to differ by at least $\Delta L_1 \geq 4$ dB from the level of the preceding level pair $\{L_1^{(2)}, L_2^{(2)}\}$ when $p_{ap,I}^{(2)} - p_{dp,I}^{(1)} > 0$; on the other hand, the level of a subsequent

level pair $\{L_1^{(3)}, L_2^{(3)}\}$ is adjusted to differ by at least $\Delta L_1 \leq -4$ dB from the level of the first level pair $\{L_1^{(1)}, L_2^{(1)}\}$, when $p_{dp,I}^{(2)} - p_{dp,I}^{(1)} \leq 0$.

9. Method according to any one of the above claims, **characterized in that**, when the first level pair $\{L_1^{(1)}, L_2^{(1)}\}$ does not produce any DPOAEs with $p_{dp,I}^{(1)}$ that have a signal-to-noise ratio of $\geq$ 4 dB, preferably $\geq$ 10 dB, the same search direction is continued until either the maximum or minimum stimulation level $L_1^{(i)}$ is reached, or a group of three valid DPOAEs with $p_{dp,I}^{(i..i+2)}$ was produced that have each a signal-to-noise ratio of $\geq$ 4 dB, preferably $\geq$ 10dB.

10. Method according to any one of the above claims, **characterized in that**, when a group of three valid DPOAEs that have a signal-to-noise ratio of $\geq$4 dB, and preferably $\geq$10 dB is not produced in the first subsequence, another subsequence is started with a higher level pair $\{L_1^{(i+1)}, L_2^{(i+1)}\}$, wherein the start level pair for the new subsequence is set to $L_2^{(i+3)} = L_2^{(1)} + 20 \pm 10$ dB, $L_1^{(i+3)} = L_1^{(1)} + 20 \pm 10$ dB, or at most to the maximum achievable level.

11. Method according to any one of the above claims, **characterized in that**, after detecting the DPOAEs of at least 3 level pairs $\{L_1^{(1..3)}, L_2^{(1..3)}\}$, preferably of one subsequence, the position of the ridge $\{L_1^{(G)}, L_2^{(G)}\}$ along the line formed by the three level pairs is determined from these three level pairs $\{L_1^{(1..3)}, L_2^{(1..3)}\}$, and a fourth level pair $\{L_1^{(4)}, L_2^{(4)}\}$ is presented that is placed at a predetermined distance down the ridge, wherein the group average of the ridge direction $\phi$ is used, and wherein a slope of the linear ridge of the level map is calculated using the DPOAEs determined from the four presented level pairs $\{L_1^{(1..4)}, L_2^{(1..4)})$.

12. Method according to claim 1 or 11, **characterized in that**, when a group of three valid DPOAEs with $p_{dp,I}^{i-2..i}$ is produced in the first or second subsequence that each have a signal-to-noise ratio of >=4 dB, preferably >=10 dB, the level pair below the ridge $\{L_1^{(G)}, L_2^{(G)}\} = L_1^{(i-2)} + \varepsilon \cdot \Delta L_1, L_2^{(i-2)} + \varepsilon \cdot \Delta L_2\}$ is determined by adapting a mathematical function to the associated DPOAEs $p_{dp,I}^{i-2...i}$, wherein $\varepsilon$ must be calculated so that $p_{dp,I}(L_1^{(G)}, L_2^{(G)})$ forms a maximum, and on that basis a fourth level pair $L_1^{(i+1)}, L_2^{(i+1)}$ is presented, with a function $\{L_1^{(i+1)}, L_2^{(i+1)}\} = \{L_1^{(i)} + \Delta L_1, L_2^{(i)} + \Delta L_2\}$, wherein $\Delta L_2 = -15 \pm 10$ dB is adjusted, and the level pair is preferably adjusted to the projection of the anticipated ridge on the $L_1, L_2$ level, i.e., adjusted with $\Delta L_1/\Delta L_2 \approx 0.51 \pm 0.15$, and wherein a slope m of the approximately linear ridge of the level map is determined using the DPOAE calculated from the four presented level pairs $L_1^{(i-2...i+1)}, L_2^{(i-2...i+1)}$.

13. Method according to any one of the above claims, **characterized in that** the level pairs $\{L_1^{(1..n)}, L_2^{(1..n)}\}$ are presented as pulsed, wherein each individual pulse is presented with a duration $T_D$ of 2 to 40 ms.

14. Method according to claim 13, **characterized in that** the level pairs $\{L_1^{(1..n)}, L_2^{(1..n)}\}$ are presented within a measuring block consisting of a plurality of level pairs $\{L_1^{(1..n,m)}, L_2^{(1..n,m)}\}$ which are presented sequentially over time in pulses, wherein level pairs $\{L_1^{(1..n,i)}, L_2^{(1..n,i)}\}$; $\{L_1^{(1..n,i+1)}, L_2^{(1..n,i+1)}\}$ that follow each other directly in time have different stimulation frequencies $\{f_{2,i}, f_{1,i}\}$; $\{f_{2,i+1}, f_{1,i+1}\}$, wherein, in particular, in an additional method step, the determined individual function of a DPOAE level map and its parameters are stored by the computer unit (10) in a non-volatile memory (16).

15. A system configured for performing the method according to any one of the above claims 1 to 14, with a computer unit (10), a main memory (15), a non-volatile memory (16) for storing a model function $p_{dp,M} = f(L_1, L_2)$ and model parameters of the model function, at least one tone output means (21, 22) controlled by the computer unit (10) for presenting tones to an individual, with at least one tone recording means (23) connected to the computer unit (10) for detecting DPOAEs from the ear of the individual, wherein in particular the at least one tone output means (21, 22) is a speaker with a highly linear characteristic and/or wherein in particular an output means (11) is provided for outputting the individual function of a DPOAE level map to a user.

## Revendications

1. Procédé pour la détermination automatique d'une fonction individuelle d'une carte de niveaux d'EOAPD avec $p_{dp,I} = f$ $(L_1, L_2)$ d'une ouïe humaine ou animale, dans laquelle des émissions oto-acoustiques de produits de distorsion (EOAPD) sont représentées sous la forme de la pression acoustique p en fonction des niveaux $L_1$ et $L_2$ des deux sons primaires utilisés pour la production des EOAPD, **caractérisé en ce qu'**il implique les étapes suivantes :

- importation (110) d'une fonction modèle (70) $p_{dp,M} = f(L_1, L_2)$ avec des paramètres modèles d'une carte de niveaux d'EOAPD, sur la base d'un nombre de N mesures d'EOAPD d'une paire de fréquences d'excitation $f_1, f_2,$ avec respectivement différentes paires de niveaux $\{L_1^{(1...N)}, L_2^{(1...N)}\}$ dans une population (p) de normo-entendants, dans une mémoire de travail (15) d'une unité de calcul (10), dans lequel $N \geq 40$ et $p \geq 2$,
- présentation automatique (120) de n différentes paires de niveaux $\{L_1^{(1..n)}, L_2^{(1...n)}\}$ d'une paire de fréquences d'excitation $f_1$, $f_2$ par le biais de moyens d'émission en sortie de sons (21, 22) à un individu et enregistrement des EOAPD correspondantes de l'individu par le biais de moyens de réception de sons (23), dans lequel au moins la première paire de niveaux $\{L_1^{(1)}, L_2^{(1)}\}$ est prédéfinie et dans lequel n << N,
- adaptation itérative (130) de la fonction modèle $p_{dp,M} = f(L_1, L_2)$ aux n EOAPD mesurées jusqu'à l'obtention d'une fonction individuelle $p_{dp,I} = f(L_1, L_2)$ avec des paramètres individuels d'une carte de niveaux d'EOAPD de l'individu par l'intermédiaire de l'unité de calcul (10),
- émission en sortie (140) de la fonction individuelle $p_{dp,I} = f(L_1, L_2)$ et/ou de ses paramètres individuels au niveau d'un moyen d'émission en sortie (11) de l'unité de calcul (10),

le procédé étant **caractérisé en ce que** la fonction modèle comprend une crête (73) croissant de manière approximativement linéaire, à laquelle sont associées des paires de niveaux $\{L_1^{(G)}, L_2^{(G)}\}$ liées de manière approximativement linéaire l'une à l'autre, dans lequel au moins la moitié des paires de niveaux $\{L_1, L_2\}$ mesurées sont situées à au moins 5 dB de distance des deux côtés du groupe des paires de niveaux $\{L_1^{(G)}, L_2^{(G)}\}$ associées à la crête (73).

2. Procédé selon la revendication 1, **caractérisé en ce que** la première paire de niveaux $\{L_1^{(1)}, L_2^{(1)}\}$ comprend un niveau $L_1^{(1)}$ de $67 \pm 10$ dB et un niveau $L_2^{(1)}$ de $57 \pm 10$ dB.

3. Procédé selon l'une des revendications précédentes, **caractérisé en ce que** les différentes paires de niveaux $\{L_1, L_2\}$ sont présentées en une séquence qui est identique pour chaque individu.

4. Procédé selon l'une des revendications précédentes, **caractérisé en ce que** les différentes paires de niveaux $\{L_1, L_2\}$ prédéfinies sont présentées en une séquence qui comprend un nombre de k sous-séquences dont les paires de niveaux $\{L_1, L_2\}$ sont situées de manière sensiblement perpendiculaire aux paires de niveaux liées de manière linéaire l'une à l'autre et associées à la crête $\{L_1^{(G)}, L_2^{(G)}\}$.

5. Procédé selon l'une des revendications précédentes, **caractérisé en ce que** $n \geq 5$ et $\leq 12$, de préférence $n \geq 5$ et $\leq 8$.

6. Procédé selon l'une des revendications précédentes, **caractérisé en ce que** $k \geq 2$ et $\leq 8$.

7. Procédé selon l'une des revendications précédentes, **caractérisé en ce que** les paires de niveaux $\{L_1^{(2..n_k)}, L_2^{(2..n_k)}\}$ suivant une première paire de niveaux $\{L_1^{(1)}, L_2^{(1)}\}$ prédéfinie d'une sous-séquence sont déterminées avec $n_k$ mesures par le biais d'une fonction $\{L_1^{(i)}, L_2^{(i)}\} = \{L_1^{(i-1)} + \mu \cdot \Delta L_1, L_2^{(i-1)} + \mu \cdot \Delta L_2\}$ à partir de la paire de niveaux respectivement précédente $\{L_1^{(i-1)}, L_2^{(i-1)}\}$, dans lequel $\mu = \pm 1$, de préférence est égal à +1, et $\Delta L_1$, $\Delta L_2$ est une distance de niveaux entre deux paires de niveaux successives et présente des valeurs allant de $\Delta L_1 = 4$ à 14 dB, de préférence de 6 à 10 dB, et $\Delta L_2 = 0$ à -2,78 dB, de préférence $\Delta L_2 = $ -1,52 à -2,78 dB.

8. Procédé selon l'une des revendications précédentes, **caractérisé en ce que**, lorsque la première paire de niveaux $\{L_1^{(1)}, L_2^{(1)}\}$ et la deuxième paire de niveaux $\{L_1^{(2)}, L_2^{(2)}\}$ produisent deux EOAPD avec $p_{dp,I}^{(12)}$ qui présentent chacune un rapport signal sur bruit >= 4 *dB,* de préférence >= 10 *dB,* le niveau d'une troisième paire de niveaux suivante $\{L_1^{(3)}, L_2^{(3)}\}$ est ajusté pour différer du niveau de la paire de niveaux précédente $\{L_1^{(2)}, L_2^{(2)}\}$ d'au moins $\Delta L_1 \geq$ 4 *dB* lorsque $p_{ap,I}^{(2)} - p_{dp,I}^{(1)} > 0$, d'autre part le niveau d'une paire de niveaux suivante $\{L_1^{(3)}, L_2^{(3)}\}$ est ajusté pour différer du niveau de la première paire de niveaux $\{L_1^{(1)}, L_2^{(1)}\}$ d'au moins $\Delta L_1 \leq$ -4 dB lorsque $p_{ap,I}^{(2)} - p_{dp,I}^{(1)} \leq 0$.

9. Procédé selon l'une des revendications précédentes, **caractérisé en ce que**, lorsque la première paire de niveaux $\{L_1^{(1)}, L_2^{(1)}\}$ ne produit pas d'EOAPD avec $p_{dp,I}^{(1)}$ qui présente un rapport signal sur bruit $\geq 4$ *dB,* de préférence $\geq 10$ *dB,* la même direction de recherche est poursuivie jusqu'à ce que soit atteint soit le niveau d'excitation maximal, soit le niveau d'excitation minimal $L_1^{(i)}$, ou que soit produit un groupe de trois EOAPD valides avec $p_{dp,I}^{(i..i+2)}$ qui présentent chacune un rapport signal sur bruit $\geq 4$ *dB,* de préférence $\geq 10$ *dB.*

10. Procédé selon l'une des revendications précédentes, **caractérisé en ce que**, lorsqu'aucun groupe de trois EOAPD valides qui présentent chacune un rapport signal sur bruit $\geq 4$ dB, de préférence $\geq 10$ dB n'est produit dans la première sous-séquence, une nouvelle sous-séquence est débutée avec une paire de niveaux supérieure $\{L_1^{(i+1)}, L_2^{(i+1)}\}$, dans lequel la paire de niveaux de départ pour la nouvelle sous-séquence est ajustée à $L_2^{(i+3)} = L_2^{(1)} + 20 \pm 10$ *dB,* $L_1^{(i+3)} = L_1^{(1)} + 20 \pm 10$ *dB,* ou au plus au niveau atteignable maximal.

**11.** Procédé selon l'une des revendications précédentes, **caractérisé en ce que**, après l'enregistrement des EOAPD d'au moins 3 paires de niveaux $\{L_1^{(1..3)}, L_2^{(1..3)}\}$, de préférence d'une sous-séquence, la position de la crête $\{L_1^{(G)}, L_2^{(G)}\}$ le long de la ligne formée par les 3 paires de niveaux est déterminée à partir de ces 3 paires de niveaux $\{L_1^{(1..3)}, L_2^{(1..3)}\}$ et une quatrième paire de niveaux $\{L_1^{(4)}, L_2^{(4)}\}$ est présentée, laquelle est placée à une distance prédéfinie vers le bas de la crête, dans lequel la valeur moyenne de groupe de la direction de crête, $\varphi$, est utilisée, et dans lequel une pente de la crête linéaire de la carte de niveaux est déterminée à l'aide des EOAPD déterminées à partir des quatre paires de niveaux présentées $\{L_1^{(1..4)}, L_2^{(1..4)}\}$.

**12.** Procédé selon la revendication 1 ou la revendication 11, **caractérisé en ce que**, lorsqu'il est produit dans la première ou deuxième sous-séquence un groupe de trois EOAPD valides avec $p_{dp,}{}^{j-2...i}$ qui présentent chacune un rapport signal sur bruit >= 4 dB, de préférence >= 10 dB, la paire de niveaux au-dessous de la crête $\{L_1^{(G)}, L_2^{(G)}\} = \{L_1^{(i-2)} + \varepsilon \cdot \Delta L_1, L_2^{(i-2)} + \varepsilon \cdot \Delta L_2\}$ est déterminée par adaptation d'une fonction mathématique à l'EOAPD correspondante $p_{dp,}{}^{j-2...i}$, dans lequel $\varepsilon$ doit être calculé de sorte que $p_{dp, l}(L_1^{(G)}, L_2^{(G)})$ forme un maximum, et sur cette base une quatrième paire de niveaux $L_1^{(i+1)}, L_2^{(i+1)}$ est présentée, avec une fonction $\{L_1^{(i+1)}, L_2^{(i+1)}\} = \{L_1^{(i)} + \Delta L_1, L_2^{(i)} + \Delta L_2\}$, dans lequel $\Delta L_2 = -15 \pm 10\ dB$ est ajustée, et la paire de niveaux est ajustée de préférence à la saillie de la crête attendue sur le plan $L_1, L_2$, c'est-à-dire avec $\Delta L_1/\Delta L_2 \approx 0{,}51 \pm 0{,}15$, et dans lequel une pente m de la crête approximativement linéaire de la carte de niveaux est déterminée à l'aide des EOAPD déterminées à partir des quatre paires de niveaux présentées $L_1^{(i-2...i+1)}, L_2^{(i-2...i+1)}$.

**13.** Procédé selon l'une des revendications précédentes, **caractérisé en ce que** les paires de niveaux $\{L_1^{(1..n)}, L_2^{(1..n)}\}$ sont présentées pulsées, dans lequel chaque impulsion individuelle est présentée avec une durée $T_D$ allant de 2 à 40 ms.

**14.** Procédé selon la revendication 13, **caractérisé en ce que** les paires de niveaux $\{L_1^{(1..n)}, L_2^{(1..n)}\}$ sont présentées au sein d'un bloc de mesure constitué de plusieurs paires de niveaux $\{L_1^{(1..n,m)}, L_2^{(1..n,m)}\}$ présentées les unes après les autres dans le temps en impulsions, dans lequel des paires de niveaux directement successives dans le temps $\{L_1^{(1..n,i)}, L_2^{(1..n,i)}\}$ ; $\{L_1^{(1..n,i+1)}, L_2^{(1..n,i+1)}\}$ présentent des fréquences d'excitation différentes $\{f_{2,i}, f_{1,i}\}$ ; $\{f_{2,i+1}, f_{1,i+1}\}$, dans lequel, en particulier dans une étape de procédé supplémentaire, la fonction individuelle déterminée d'une carte de niveaux d'EOAPD et de ses paramètres est sauvegardée par l'unité de calcul (10) dans une mémoire non volatile (16).

**15.** Système (1) conçu pour la mise en œuvre du procédé selon l'une des revendications 1 à 14, avec une unité de calcul (10), avec une mémoire de travail (15), avec une mémoire non volatile (16) pour la mémorisation d'une fonction modèle $p_{dpM} = f(L_1, L_2)$ et de paramètres modèles de la fonction modèle, avec au moins un moyen d'émission en sortie de sons (21, 22) commandé par le biais de l'unité de calcul (10) pour la présentation de sons à un individu, avec au moins un moyen de réception de sons (23) relié à l'unité de calcul (10) pour l'enregistrement d'EOAPD issues de l'oreille de l'individu, dans lequel en particulier l'au moins un moyen d'émission en sortie de sons (21, 22) est un haut-parleur avec une caractéristique hautement linéaire et/ou dans lequel en particulier un moyen d'émission en sortie de sons (11) est prévu pour l'émission en sortie de la fonction individuelle d'une carte de niveaux d'EOAPD vers un utilisateur.

Fig. 1

Fig. 2

```
┌─────────────────────────────────────────────┐
│                                             │ ⟋ 110
│         Einlesen der Modellfunktion         │
│                                             │
└─────────────────────────────────────────────┘
                      │
                      ▼
┌ ─ ─ ─ ─ ─ ─ ─ ─ ─ ─ ─ ─ ─ ─ ─ ─ ─ ─ ─ ─ ─ ┐  ⟋ IV
│ ┌─────────────────────────────────────────┐ │
│ │                                         │ │ ⟋ 120
│ │  Präsentation von n Pegelpaaren und     │ │
│ │  Erfassung der zugehörigen              │ │
│ │  Mikrophonsignale                       │ │
│ └─────────────────────────────────────────┘ │
└ ─ ─ ─ ─ ─ ─ ─ ─ ─ ─ ─ ─ ─ ─ ─ ─ ─ ─ ─ ─ ─ ┘
                      │
                      ▼
┌─────────────────────────────────────────────┐
│                                             │ ⟋ 130
│  Anpassung der Modellfunktion an die        │
│  Messwerte                                  │
│                                             │
└─────────────────────────────────────────────┘
                      │
                      ▼
┌─────────────────────────────────────────────┐
│                                             │ ⟋ 140
│  Ausgabe der individuell angepaßten         │
│  Modellfunktion und ihrer                   │
│  Funktionsparameter                         │
└─────────────────────────────────────────────┘
```

Fig. 3

Einlesen Startpegel: $L_1^{(1)}$, $L_2^{(1)}$, sowie Schwellen für Suchrichtungsentscheidungen ($L_{dp,,min}^{(G,1)}$; $SNR_{min}'$ ) und Schrittweiten $\Delta L_1'$, $\Delta L_2'$. — 121

Messung 1. Subsequenz quer zum vermuteten Grat: Variation von $\Delta L_1'$ in $L_1^{(n+1)}$ = $L_1^{(n)}$ + $\Delta L_1'$ cos($\varphi$) und $L_2^{(n+1)}$ = $L_2^{(n)}$ - $\Delta L_1'$ sin($\varphi$) ; wird absteigende oder keine Flanke gemessen, wird Suchrichtung umgekehrt: $\Delta L_1'$ =- $\Delta L_1'$ . — 122

| Wurden drei valide DPOAE gemessen? | |
|---|---|
| nein | ja |
| Wiederholung mit $L_2^{(1)} = L_2^{(alt)} + \Delta L_2'$ cos($\varphi$) , $L_1^{(1)} = L_1^{(alt)} + \Delta L_2'$ sin($\varphi$) | |

— 123

Bestimmung der Lage des Grates anhand der drei Messwerte, z.B. durch Lösung einer Parabelgleichung und Auffinden des individuellen Maximums: $L_{dp}^{(G,1)} = f (L_1^{(G,1)}, L_2^{(G,1)})$ — 124

Messung 2. Subsequenz entlang des vermuteten Grates (ein Messwert): $L_1^{(4)}$ = $L_1^{(G,1)}$ - $\Delta L_2'$ sin($\varphi$) und $L_2^{(4)}$ = $L_2^{(G,1)}$ - $\Delta L_2'$ cos($\varphi$) ; unterschreitet $L_{dp}^{(G,1)}$ eine voreingestellte Grenze ($L_{dp,min}^{(G,1)}$), wird dieser Schritt zu höheren Pegeln hin ausgeführt ($\Delta L_2'$ =- $\Delta L_2'$). — 125

Berechnung der individuellen Steigung des Grates, $m=f(L_{dp}^{(G,1)}, L_1^{(G,1)}, L_2^{(G,1)}, L_{dp}^{(4)}, L_1^{(4)}, L_2^{(4)})$, Festlegung eines optimalen Startpegels $L_1^{(5)}$, $L_2^{(5)}$ für die 3. Subsequenz — 126

Messung 3. Subsequenz quer zum vermuteten Grat: Variation von $\Delta L_1'$ in $L_1^{(n+1)}$ = $L_1^{(n)}$ + $\Delta L_1'$ cos($\varphi$) und $L_2^{(n+1)}$ = $L_2^{(n)}$ - $\Delta L_1'$ sin($\varphi$). — 127

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014108663
- DE 102014108663 A1

**In der Beschreibung aufgeführte Nicht-Patentliteratur**

- **B. P. KIMBERLEY** ; **D. A. NELSON.** *J. Otolaryngol.*, December 1989, vol. 18 (7), 365-369
- **D. A. NELSON** ; **B. P. KIMBERLEY**. *J.Speech Hear.Res.*, October 1992, vol. 35 (5), 1142-1159
- **P. BOEGE** ; **T. JANSSEN**. *J. Acoust. Soc. Am.*, April 2002, vol. 111 (4), 1810-1818
- **P. KUMMER** ; **T. JANSSEN** ; **P. HULIN** ; **W. ARNOLD**. Optimal L1-L2 primary tone level separation remains independent of test frequency in humans. *Hear.Res.*, August 2000, vol. 146 (1-2), 47-56
- **M. P. GORGA**. *J. Acoust. Soc. Am.*, June 2003, vol. 113 (6), 3275-3284
- **N. SCHMUZIGER**. *J. Acoust. Soc. Am.*, April 2006, vol. 119 (4), 1937-1939
- **E. DALHOFF** ; **A. VETESNIK** ; **D. TURCANU** ; **A. W. GUMMER**. Schall- und Geschwindigkeits-DPOAE: Technologie, Methodik und Perspektiven. *HNO*, June 2010, vol. 58 (6), 543-555
- **D. ZELLE** ; **A. W. GUMMER** ; **E. DALHOFF**. Extraction of otoacoustic distortion product sources using pulse basis functions. *J.Acoust.Soc.Am.*, July 2013, vol. 134 (1), EL64-EL69
- **M. L. WHITEHEAD** ; **B. B. STAGNER** ; **M. J. MCCOY** ; **B. L. LONSBURY-MARTIN** ; **G. K. MARTIN**. Dependence of distortion-product otoacoustic emissions on primary levels in normal and impaired ears. II. Asymmetry in L 1 , L 2 space. *The Journal of the Acoustical Society of America*, 1995, vol. 97 (4)
- **P. BOEGE** ; **T. JANSSEN**. *J. Acoust. Soc. Am.*, 2002, vol. 111 (4), 1810-1818